# EUROPEAN PATENT APPLICATION

(11) **EP 2 924 028 A1**
(43) Date of publication of application: **30.09.2015**
(21) Application number: 13857010.6
(22) Date of filing: 21.11.2013
(51) Int. Cl.: C07D 285/135, A61K 31/433, A61P 29/02

(54) **TRPV-1 RECEPTOR ANTAGONIST COMPOUND DERIVED FROM 1,3,4-THIADIAZOLE ALKYLAMIDES AND CHALCONES**

(30) Priority: 22.11.2012 CL 2012003253
(71) Applicant: Universidad De Concepcion, Santiago (CL)
(72) Inventor: ZÁRRAGA OLAVARRIA, Miguel, Concepción (CL); BRAUCHI ULLOA, Sebastián, Concepción (CL); LESPAY REBOLLEDO, Carolyne, Concepción (CL)
(74) Representative: Elzaburu S.L.P.
(86) International application number: PCT/CL2013/000085
(87) International publication number: WO 2014/078971

(57) **Abstract**

This technology encompasses compounds derived from 1, 3, 4-thiadiazole alkylamides and chalcone, which inhibit the activation of the TRPV-1 receptor using capsaicin and temperature. Also disclosed is the use of these compounds in the treatment of diseases with TRPV-1 overexpression, such as chronic pain.

## Description

### Field of the Invention

The present technology is oriented towards the pharmaceutical sector, mainly for the production of TRPV1 receptor antagonist pharmaceuticals, in order to treat diseases where the said receptor is over-activated, for example chronic pain.

### State of the Art

A target for the development of drugs for treating chronic inflammatory pain is the transient receptor potential vanilloid 1 receptor or TRPV1, as it is related to the modulation of nociceptive signals due to its expression mainly in C and Aδ sensory fibers (1, 2). Under pathological conditions, the said receptors are over-activated in these sensory fibers and have a direct role in maintaining neurogenic inflammation (3, 4, 5, 6). In addition, they represent a possible treatment for neuropathic pain due to the overexpression of these receptors in undamaged fibers adjacent to the damaged fibers.

TRPV1 receptors are calcium permeable non-selective cation channels known as polymodal nociceptors, as they can be activated by various physical and chemical ligands, both endogenous and exogenous. Direct activators of TRPV1 receptors that have been discovered to date are endovanilloids (anandamide, arachidonic acid or lipoxygenase products), basic or acid pH, harmful temperatures (>42 °C) and voltage (8, 9), in addition to exogenous chemical ligands such as capsaicin. However, TRPV1 receptors can be indirectly activated or sensitized by changes in the phosphorylation-dephosphorylation state produced by kinases, which are activated through secondary pathways as a result of the activation of receptors, e.g. bradykinin, serotonin, histamine, somatostatin, prostaglandin, interleukin or tyrosinase receptors, so that the activation of these receptors finally leads to the activation of the TRPV1 receptors (3).

The receptors related to the indirect activation of TRPV1 receptors are activated by pro-inflammatory molecules liberated at the site of tissue inflammation in pathologies associated with inflammatory mechanisms, such as cancer, osteoporosis, arthritis, diabetes, irritable bowel syndrome, cystitis and others (10, 11). Some of these pro-inflammatory molecules can also directly activate TRPV1 receptors, e.g. anandamide, arachidonic acid, N-arachidonoyl-dopamine, N-acyl-dopamine, 12-hydroperoxyeicosatetraenoic acid, 15-hydroperoxyeicosatetraenoic acid and leukotriene B4, which have been shown to effectively activate TRPV1 receptors (12). TRPV1 receptors are therefore directly involved in the perception and transduction of pain.

The creation of TRPV1 knockout mice has made it possible to confirm the principal role played by the activation of the receptor in nociceptive processes and its potential for treating chronic pain diseases. These mice lacking the TRPV1 receptor present an impaired physiological response to vanilloid compounds and, in addition, neuron cultures from the dorsal root ganglia (DRG) of knockout mice do not respond to typical activators of the receptor, such as capsaicin, damaging heat and protons; furthermore, these mice are characterized for not developing thermal hyperalgesia and mechanical allodynia, two recurring symptoms in chronic pain patients (13, 14, 15).

In addition to the inhibition of TRPV1 receptors for treating pain, the activation of TRPV1 receptors can also be modulated to treat pathologies such as urinary incontinence, overactive bladder syndrome, asthma, chronic cough, renal hypertension, ischemic neuronal protection, pancreatitis or obesity, as the expression of TRPV1 receptors is also present in non-sensory cells, such as keratinocytes, pancreatic β cells and dendritic cells, among other cellular phenotypes (11), thus expanding pharmacological possibilities with regard to these receptors.

The first ligand discovered for TRPV1 receptors was capsaicin, a pungent compound found in chili peppers that acts as a TRPV1 agonist and whose therapeutic potential lies in its ability to sensitize DRG sensory neurons and cause a desensitizing effect of these neurons after exposure to capsaicin. The response of DRG neurons is characterized by an acute desensitization or tachyphylaxis, depending on the time of the amount of repetitive doses of capsaicin, respectively, and both desensitization mechanisms are dependent on extracellular calcium (16). The effect produced by capsaicin thus alters the transduction of nociceptive signals and is the basis for capsaicin's analgesic properties in topical applications in patients with chronic pain (17, 18).

A group of natural compounds known as vainilloids are characterized for presenting agonist activity on TRPV1 receptors, e.g. resiniferatoxin, polygodial, isovaleral, scutigeral, neogrifolin, gingerol, eugenol and piperine derivatives. These compounds are structural analogs of capsaicin and their synthesis is relevant for the development of drugs due to the fact that capsaicin acts specifically on TRPV1 receptors and causes specific desensitization in DRG C and Aδ fibers (19, 20). The agonist activity of vanilloid compounds can be modified through halogenation in carbon atoms 5 and 6 of the vanillyl group, changing the effect to an antagonistic one, where the chlorine and bromide atoms in these positions reduce agonistic activity, and the iodine atom completely reverts the activity, so that 5/6-iodononivamide and 5-iodoresiniferatoxin are characterized for acting as complete TRPV1 antagonists with a half maximal inhibitory concentration (IC₅₀) of 10; 126.2 and 0.4 nM, respectively; of which resiniferatoxin, a daphnane diterpene isolated from *Euphorbia resinifera,* is additionally an agonist that is 10 times more potent than capsaicin and its potency is unaffected by its iodation, although its pharmacological development is limited by its low availability and high toxicity. The antagonistic effect exerted by the iodine atom can also be observed in other iodinated vanilloid analogs (21, 22, 23).

The development of compounds with antagonistic activity on TRPV1 receptors, both capsaicin analogs and other structural classes of antagonists with a specific activity, is an emerging area of pharmaceutical research aimed at treating chronic pain and other diseases in which the activity of these receptors is increased. This is mainly due to the fact that treatments based on agonists have disadvantages such as neurotoxicity associated with the increase in intracellular calcium, and although capsaicin is effective in chronic pain patients its topical application in humans induces initial pain, erythema and hyperalgesia and it must be applied repeatedly to achieve an analgesic effect due to the low concentrations of capsaicin in formulations in order to reduce the side effects, which together with making it unsuitable for prolonged use leads to a high number of patients abandoning topical treatments based on capsaicin. In addition, the pungency and systemic side effects in vanilloid analogs prevent the development of agonist compounds with oral bioavailability, in addition to the existing relationship between the higher agonist potency and the presence of the hydroxyl group (3, 18).

The main competitive antagonist compounds developed until now are characterized for acting at the orthosteric site of the TRPV1 receptor located between the third and fourth transmembrane segment, where the threonine 550 and tyrosine 510 residues are crucial in the sensitivity of the receptor to both agonist and antagonist compounds (25-28). This binding site reveals two important requirements for the recognition of the receptor, these being lipophilicity for reaching the binding site and the establishment of hydrogen bonds for interacting with the residues identified, and these characteristics are consistent with the chemical modifications carried out on compounds with activity at the TRPV1 receptor that identify three pharmacophoric regions that are important for the activity, these being a donor-acceptor region of hydrogen bonds, a region with a high polarity and a hydrophobic region, which three regions are separated by spacing groups that regulate the conformation of the ligands and thus their agonist or antagonist behavior towards the receptor. The agonist compounds thus acquire a folded conformation between the donor-acceptor region of hydrogen bonds and the hydrophobic region, in which the capsaicin amide group acquires, for example, a trans-orientation and the aliphatic chain acquires an extended conformation (29). At the same time, the antagonist compounds adopt a coplanar conformation between the hydrophobic region and the donor-acceptor region of hydrogen bonds (30).

Based on these structural and conformational criteria, the most potent antagonists, such as cinnamide, pyrimidine and quinazoline derivatives, among many others (31-37), are characterized for maintaining these characteristics and also by presenting a high conformational restriction imposed between the polar and the hydrophobic region, which favors the coplanar rearrangement between the hydrophobic region and the donor-acceptor region of hydrogen bonds, improving antagonistic potency. Thus, the compound N-(4-tertiarybutylphenyl)-4-(3-cholorphyridin-2-yl)tetrahydropyrazine-1 (2H)-carbox-amide known as BCTC is characterized for being a conformationally-restricted urea derivative with potent TRPV1 antagonistic qualities and a half maximal inhibitory concentration (IC₅₀) of 2-6nM depending on the type of species (rat, mouse or human) of the TRPV1 receptor, and although its efficacy has been observed in chronic pain animal models, it has a low degree of metabolic stability, a short half-life, poor aqueous solubility and only moderate oral bioavailability (31, 32, 33). Similarly, the cinnamide derivative (E)-3-(4-t-butylphenyl)-N-(2,3-dihydrobenzo[b][1,4]dioxin-6-yl)acrylamide presents antagonistic activity with IC₅₀ 79nm, and replacing the double bond in this compound with a 4-Aminopyridine group made it possible to obtain a derivative with increased antagonistic activity with an IC₅₀ of 2nM (34). Additionally, the synthesis of oxazole derivatives was developed based on urea derivatives such as ABT-102, where the isosteric replacement of the urea group resulted in compounds with strong antagonistic activity and led to the obtainment of compounds with improved pharmacokinetic properties and good CNS penetration (41). Thus, isosteric groups, such as imidazole, thiazolpyrimidine, pyridopyrimidines, pyridopyrazines or thiazole carboxamides have made it possible to obtain a wide range of conformationally-restricted competitive antagonists with strong activity towards TRPV1 receptors (31-44).

In addition to competitive antagonists, non-competitive antagonists have been characterized by TRPV1, such as ruthenium red with an antagonistic activity of IC₅₀ = 0.17µM and lanthanum which presents activity at a concentration equal to or greater than 100µM, which act by blocking the ionic channel, binding to a site composed of negatively-charged residues; arginine-rich hexapeptides have also been developed, although these compounds together with ruthenium red and lanthanum lack selectivity for the receptor, resulting in side effects and toxicity and preventing their pharmacological development (45, 46).

### References

1.- Michael J. Caterina, Mark A. Schumacher, Makoto Tominaga, Tobias A. Rosen, Jon D. Levine and David Julius. The capsaicin receptor: a heat-activated ion channel in the pain pathway. Nature, 1997, 389, 816-824.
2.- Kendall Mitchell, Brian D Bates, Jason M Keller, Matthew Lopez, Lindsey Scholl, Julia Navarro, Nicholas Madian, Gal Haspel, Michael I Nemenov and Michael Jladarola. Ablation of rat TRPV1-expressing Adelta/C-fibers with resiniferatoxin: analysis of withdrawal behaviors, recovery of function and molecular correlates. Molecular Pain, 2010, 6, 1-13.
3.- Marcello Trevisani, and Arpad Szallasi. Targeting TRPV1: Challenges and Issues in Pain Management. The Open Drug Discovery Journal, 2010, 2, 37-49.
4.- Andres Jara-Oseguera, Sidney A. Simon, and Tamara Rosenbaum. TRPV 1: on the road to pain relief. Curr Mol Pharmacol. 2008, 1, 255-269.
5.- Louise A. Roberts and Mark Connor. TRPV1 Antagonists as a Potential Treatment for Hyperalgesia. Recent Patents on CNS Drug Discovery, 2006, 1, 65-76.
6.- Gilbert Y. Wonga, Narender R. Gavva. Therapeutic potential of vanilloid receptor TRPV1 agonists and antagonists as analgesics: Recent advances and setbacks. Brain Research Reviews, 2009, 60, 267-277.
7.-Enza Palazzo, Livio Luongo, Vito de Novellis, Liberato Berrino, Francesco Rossi and Sabatino Maione. Moving towards supraspinal TRPV1 receptors for chronic pain relief. Molecular Pain. 2010, 6, 1-11
8.-Ramon Latorre, Cristián Zaelzer and Sebastian Brauchi. Structure-functional intimacies of transient receptor potential channels. Quarterly Reviews of Biophysics, 2009, 42, 201-246.
9.- Andres Jara-Oseguera, Andrés Nieto-Posadas, Arpad Szallasi, Leon D. Islas and Tamara Rosenbaum. Molecular Mechanisms of TRPV1 Channel Activation. The Open Pain Journal, 2010, 3, 68-81
10.- Bernd Nilius, Grzegorz Owsianik, Thomas Voets, and John a. Peters. Transient Receptor Potential Cation Channels in Disease. Physiology Review, 2007, 87, 165-217.
11.- Khadija Alawi and Julie Keeble. The paradoxical role of the transient receptor potential vanilloid 1 receptor in inflammation. Pharmacology & Therapeutics, 2010, 125, 181 -195.
12.- Mario van der Stelt and Vincenzo Di Marzo. Endovanilloids Putative endogenous ligands of transient receptor potential vanilloid 1 channels. Eur. J. Biochem. 2004, 271,1827-1834.
13.- M. J. Caterina, A. Leffler, A. B. Malmberg, W. J. Martin, et ál. Impaired Nociception and Pain Sensation in Mice Lacking the Capsaicin Receptor. Science. 2000, 288, 306-313
14.- Lu Yu1, Fei Yang1, Hao Luo1, Feng-Yu Liu1, Ji-Sheng Han1,2,3, Guo-Gang Xing*1,2 and You Wan. The role of TRPV1 in different subtypes of dorsal root ganglion neurons in rat chronic inflammatory nociception induced by complete Freund's adjuvant. Molecular Pain, 2008, 4, 1-10.
15.- Jurgen Sandkuhler. Models and Mechanisms of Hyperalgesia and Allodynia. Physiology Review. 2009, 89, 707-758.
16.- Patricia A. Koplas, Robert L. Rosenberg, and Gerry S. Oxford. The Role of Calcium in the Desensitization of Capsaicin Responses in Rat Dorsal Root Ganglion Neurons. The Journal of Neuroscience, 1997, 173, 525-3537.
17.-Gilbert Y. Wonga,1, Narender R. Gavva. Therapeutic potential of vanilloid receptor TRPV1 agonists and antagonists as analgesics: Recent advances and setbacks. Brain Research Reviews, 2009, 60, 267-277
18.-Lorna Mason, R. Andrew Moore, Sheena Derry, Jayne E. Edwards, Henry J. McQuay. Systematic review of topical capsaicin for the treatment of chronic pain. BMJ, 2004, 328, 991-994.
19.- Rosa Planells-Cases, Carolina García-Martínez1, Miriam Royo, Enrique Pérez-Payá, Cristina Carreño, Fernando Albericio, Angel Messeguer, and Antonio Ferrer-Montiel. Small molecules targeting the vanilloid receptor complex as drugs for inflammatory pain. Drugs of the Future, 2003, 28, 1-28.
20.-Holzer, P. Capsaicin: cellular targets, mechanisms of action, and selectivity for thin sensory neurons. Pharmacological Reviews, 1991, 43, 143-201.
21.- Dong Wook Kang , Yong Soo Kim, Kwang Su Lim , Myeong Seop Kim, Larry V. Pearce, et ál. Halogenation of 4-hydroxy/amino-3-methoxyphenyl acetamide TRPV1 agonists showed enhanced antagonism to capsaicin. Bioorganic & Medicinal Chemistry. 2010, 18, 8092-8105.
22.- Kwang Su Lim, Dong Wook Kang , Yong Soo Kim, Myeong Seop Kim, Seul-Gi Park , Sun Choi, Larry V. Pearce , Peter M. Blumberg , Jeewoo Lee. Receptor activity and conformational analysis of 5-halogenatedresiniferatoxin analogs as TRPV1 ligands. Bioorganic & Medicinal Chemistry Letters. 2011, 21, 299-302.
23.- Giovanni Appendino, Nives Daddario, Alberto Minassi, Aniello,Schiano Moriello, Luciano De Petrocellis, and Vincenzo Di Marzo. The Taming of Capsaicin. Reversal of the Vanilloid Activity of N-Acylvanillamines by Aromatic Iodination. Journal of Medicinal Chemistry. 2005, 48, 4663-4669.
24.- Derek S. Reubish, Daniel E. Emerling, Jeff DeFalco, Daniel Steiger, Cheryl L. Victoria, and Fabien Vincent. Functional assessment of temperature-gated ion-channel activity using a real-time PCR machine. Short Technical Reports, 2009, 47, 3-9.
25.- Sven-Eric Jordt and David Julius. Molecular Basis for Species-Specific Sensitivity to "Hot" Chili Peppers. Cell, 2002, 108, 421-430.
26.- Narender R. Gavva, Lana Klionsky, Yusheng Qu, Licheng Shi, Rami Tamir, Steve Edenson, T. J. Zhang, Vellarkad N. Viswanadhan, Attila Toth, Larry V. Pearce, Todd W. Vanderah, Frank Porreca, Peter M. Blumberg, Jack Lile, Yax Sun, Ken Wild,Jean-Claude Louis, and James J. S. Treanor. Molecular Determinants of Vanilloid Sensitivity in TRPV1. The journal of biological chemistry, 2004, 279, 20283-20295.
27.- Margaret Z. Chou, Tecla Mtui, Ying-Duo Gao, Martin Kohler and Richard E. Middleton. Resiniferatoxin Binds to the Capsaicin Receptor (TRPV1) near the Extracellular Side of the S4 Transmembrane Domain. Biochemistry, 2004,43,2501-2511**.**
28.- Jin Hee Lee, Yoonji Lee, HyungChul Ryu, Dong Wook Kang, Jeewoo Lee, Jozsef Lazar , Larry V. Pearce, Vladimir A. Pavlyukovets, Peter M. Blumberg, Sun Choi. Structural insights into transient receptor potential vanilloide type 1 (TRPV1) from homology modeling, flexible docking and mutational studies. J Comput Aided Mol Des. 2011, 25, 317-327.
29.- Margit Winkler, Thomas Moraux, Hesham A. Khairy, et al. Synthesis and Vanilloid Receptor (TRPV-1) Activity of the Enantiomers of a-Fluorinated Capsaicin. ChemBioChem, 10, 2009, 823-828.
30.- Brian S. Brown , Ryan Keddy, Guo Zhu Zheng, Robert G. Schmidt, et al. Tetrahydropyridine-4-carboxamides as novel, potent transient receptor potential vanilloid 1 (TRPV1) antagonists. Bioorganic & Medicinal Chemistry 16, 2008, 8516-8525.
31.- Craig C. Corrella , P. Tara Phelpsa, John C. Anthesa, Shelby Umlandb, Scott Greenfeder.Cloning and pharmacological characterization of mouse TRPV1. Neuroscience Letters 370, 2004, 55-60.
32.- Qun Sun, Laykea Tafesse, Khondaker Islam. 4-(2-Pyridyl)piperazine-1-carboxamides: Potent Vanilloid Receptor 1 Antagonists. Bioorganic & Medicinal Chemistry Letters 13, 2003, 3611-3616.
33.- Vassil I. Ognyanov, Chenera Balan, Anthony W. Bannon. Design of Potent, Orally Available Antagonists of the Transient Receptor Potential Vanilloid 1. Structure-Activity Relationships of 2-Piperazin-1-yl-1 H-benzimidazoles. Journal Medicinal Chemistry, 2006, 49, 3719-3742.
34.- Mark H. Norman, Jiawang Zhu, Christopher Fotsch, Yunxin Bo, Ning Chen, Partha Chakrabarti, Elizabeth M. Doherty et ál. Novel vanilloid receptor-1 antagonists: 1. Conformationally restricted analogues of trans-cinnamides. Journal of Medicinal Chemistry, 2007, 50, 3497-3514.
35.- Elizabeth M. Doherty, Christopher Fotsch, Yunxin Bo, Partha P. et ál. Discovery of Potent, Orally Available Vanilloid Receptor-1 Antagonists. Structure-Activity Relationship of N-Aryl Cinnamides. Journal of Medicinal Chemistry, 2005, 48 , 71-90.
36.- Nuria Tamayo, Hongyu Liao, Markian M. Stec, Xianghong Wang, et ál. Design and Synthesis of Peripherally Restricted Transient Receptor Potential Vanilloid 1 (TRPV 1) Antagonists. Journal of Medicinal Chemistry, 2008, 51, 2744-2757.
37.- Michele C. Jetter, James J. McNally, Mark A. Youngman, Mark E. McDonnell, et ál. N -Pyridin-3-yl-and N -quinolin-3-yl-benzamides: Modulators of Human Vanilloid Receptor 1 (TRPV1). Bioorganic & Medicinal Chemistry Letters, 2008, 18, 2730-2734.
38.- Bin Shao, Jincheng Huang, Qun Sun, Kenneth J. Valenzano, Lori Schmid and Scott Nolan. 4-(2-Pyridyl) piperazine-1-benzimidazoles as potent TRPV1 antagonists. Bioorganic & Medicinal Chemistry Letters, 2005, 15, 719-723.
39.- Irene Drizin, Arthur Gomtsyan, Erol K. Bayburt, Robert G. Schmidt, Guo Zhu Zheng, et ál. Structure-activity studies of a novel series of 5, 6-fused heteroaromatic ureas as TRPV1 antagonists. Bioorganic & Medicinal Chemistry, 2006, 14, 4740-4749.
40.- Ronald Palin, Lynn Abernethy, Nasrin Ansari, Kenneth Cameron, Tom Clarkson, Maureen Dempster, et ál. Structure-activity studies of a novel series of isoxazole-3-carboxamide derivatives as TRPV1 antagonists. Bioorganic & Medicinal Chemistry Letters, 2011, 21, 892-898.
41.- Richard J. Perner, John R. Koenig, Stanley DiDomenico, Arthur Gomtsyan, et ál. Synthesis and biological evaluation of 5-substituted and 4, 5-disubstituted-2-arylamino oxazole TRPV1 antagonists. Bioorganic & Medicinal Chemistry, 2010,18,4821-4829.
42.- Ning Xi, Yunxin Bo, Elizabeth M. Doherty, Christopher Fotsch, et ál. Synthesis and evaluation of thiazole carboxamides as vanilloid receptor 1 (TRPV1) antagonists. Bioorganic & Medicinal Chemistry Letters, 2005, 15, 5211-5217.
43.- Kevin J. Hodgetts, Charles A. Blum, Timothy Caldwell, Rajagopal Bakthavatchalam, Xiaozhang Zheng, et al. Pyrido[2,3-b]pyrazines, discovery of TRPV1 antagonists with reduced potential for the formation of reactive metabolites. Bioorganic & Medicinal Chemistry Letters, 2010, 20, 4359-4363.
44.- Natalie A. Hawryluk, Jeffrey E. Merit, Alec D. Lebsack, Bryan J. Branstetter, Michael D. Hack, et ál. Discovery and synthesis of 6, 7, 8, 9-tetrahydro-5H-pyrimido-[4,5-d]azepines as novel TRPV1 antagonists. Bioorganic & Medicinal Chemistry Letters, 2010, 20, 7137-7141.
45.- Angel Messeguer, Rosa Planells-Cases and Antonio Ferrer-Montiel. Physiology amd Pharmacology of the Vanilloid Receptor. Current Neuropharmacology. 2006, 4, 1-15.
46.- Joris Vriens, Giovanni Appendino and Bernd Nilius. Pharmacology of Vanilloid Transient Receptor Potential Cation Channels. Molecular Pharmacology 75, 2009, 1262-1279.
47.- Christopher S. J. Walpole, Stuart Bevan, Graham Bloomfield, Robin Breckenridge, Ian F. James, Timothy Ritchie, Arpad Szallazi, Janet Winter and Roger Wrigglesworth. Analogues of capsaicin with agonist activity as novel analgesic agents; structure-activity studies. 2. The amide bond "B-region". Journal of Medicinal Chemistry, 1993, 36, 2373-2380.
48.- Jeewoo Lee, Mi-Kyoung Jin, Sang-Uk Kang and et ál. Analysis of structure-activity relationships for the 'A-region' of N-(4-t-butylbenzyl)-N'-[4-(methylsulfonylamino)benzyl]thiourea analogues as TRPV1 antagonists. Journal Bioorganic and Medicinal Chemistry, 2005, 15, 4136-4142.
49.- Young-Ger Suh, Yong-Sil Lee, Kyung-Hoon Min, Ok-Hui Park, Jin-Kwan Kim, Ho-Sun Seung,Seung-Yong Seo, Bo-Young Lee, Yeon-Hee Nam and et Al, Novel Potent Antagonists of Transient Receptor Potential Channel, Vanilloid Subfamily Member 1: Structure-Activity Relationship of 1,3-Diarylalkyl Thioureas Possessing New Vanilloid Equivalents. Journal of Medicinal Chemistry, 2005, 48, 18, 5823-5836.

### BRIEF DESCRIPTION OF THE FIGURES

**Figure 1****,** vanilloid derivatives of 1, 3, 4-thiadiazole alkylamides.
**Figure 2** and **Figure 3****,** chalcone derivatives or alpha, beta-unsaturated carbonyl derivatives.
**Figure 4****,** synthesis route used for the iodination of 4-Hydroxy-3-methoxybenzaldehyde or vanillin.
**Figure 5****,** synthesis route used to obtain the 1, 3, 4-thiadiazole-2-amino derivatives.
**Figure 6****,** synthesis route used to obtain the 1, 3, 4-thiadiazole alkylamides derivatives.
**Figure 7****,** synthesis route used to obtain the chalcone derivatives or alpha, beta-unsaturated carbonyl derivatives.
**Figure 8****,** nuclear magnetic resonance spectrum of ¹H (DMSO, 400 MHz) obtained for the compound N-[5-(4-hydroxy-3-methoxyphenyl)-1, 3, 4-thiadiazole-2-yl]-nonamide [b].
**Figure 9****,** nuclear magnetic resonance spectrum of ¹³C (DMSO, 100 MHz) obtained for the compound N-[5-(4-hydroxy-3-methoxyphenyl)-1, 3, 4-thiadiazole-2-yl]-nonamide [b].
**Figure 10****,** magnetic resonance spectrum of ¹H (DMSO, 400 MHz) obtained for the compound N-[5-(4-hydroxy-5-methoxy-3-iodophenyl)-1, 3, 4-thiadiazole-2-yl]-heptanamide [e].
**Figure 11****,** nuclear magnetic resonance spectrum of ¹³C (DMSO, 100 MHz) obtained for the compound N-[5-(4-hydroxy-5-methoxy-3-iodophenyl)-1, 3, 4-thiadiazole-2-yl]-heptanamide [e].
**Figure 12****,** magnetic resonance spectrum of ¹H (DMSO, 400 MHz) obtained for the compound *(2E)*-1-(4-bromodiphenyl)-3-(4-hydroxy-3-methoxyphenyl)prop-2-en-1-one [I].
**Figure 13****,** nuclear magnetic resonance spectrum of ¹³C (DMSO, 100 MHz) obtained for the compound *(2E)-*1-(4-bromodiphenyl)-3-(4-hydroxy-3-methoxyphenyl)prop-2-en-1-one [I].
**Figure 14****,** assay graph for receptor TRPV1 activation by capsaicin for the derivatives of 1, 3, 4- thiadiazole alkylamides assessed at a concentration of 1 µM.
**Figure 15****,** assay graph for receptor TRPV1 activation by capsaicin for the derivatives of 1, 3, 4- thiadiazole alkylamides (without capsaicin).
**Figure 16****,** assay graph for receptor TRPV1 activation by capsaicin for the chalcone derivatives.
**Figure 17****,** assay graph for receptor TRPV1 activation by capsaicin for the chalcone derivatives (without capsaicin).
**Figure 18****,** rat TRPV1 response to increases in temperature.
**Figure 19****,** assay graph for receptor TRPV1 activation by temperature for the derivatives of 1, 3, 4- thiadiazole alkylamides.
**Figure 20****,** assay graph for receptor TRPV1 activation by temperature for the derivatives of 1, 3, 4- thiadiazole alkylamides (without capsaicin).
**Figure 21****,** assay graph for receptor TRPV1 activation by temperature for the chalcone derivatives.
**Figure 22****,** assay graph for receptor TRPV1 activation by temperature for the chalcone derivatives (without capsaicin).
**Figure 23****,** dose-response curve for derivatives of 1, 3, 4- thiadiazole alkylamides in HEK 293T cells that overexpress the TRPV1 receptor.
**Figure 24****,** dose-response curve for chalcone derivatives in HEK 293T cells that overexpress the TRPV1 receptor.

### DESCRIPTION OF THE INVENTION

This invention describes compounds derived from 1,3,4-thiadiazole alkylamides and from chalcones with the capacity of inhibiting the activation of the TRPV1 receptor caused by capsaicin and temperature.

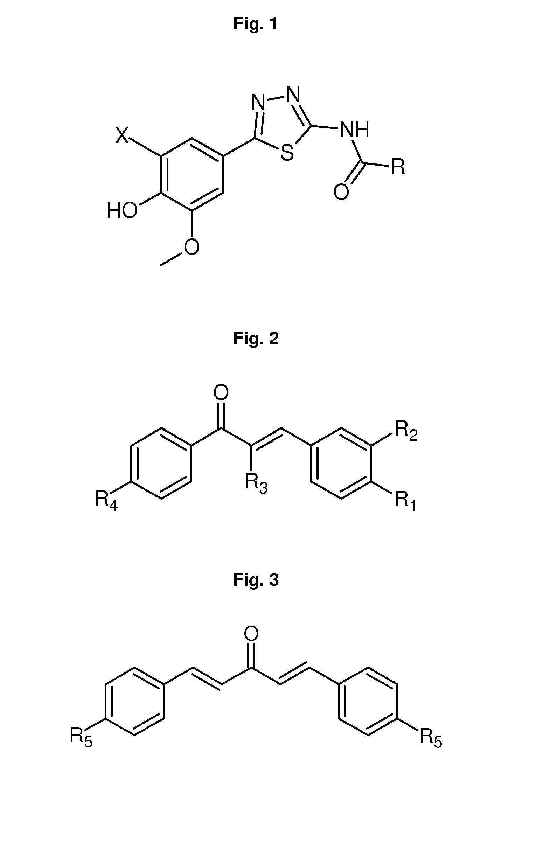

The first aspect of the invention is related to the vanilloid derivatives of 1, 3, 4-thiadiazole alkylamides described in **Figure 1****.**

Where R is a saturated alkyl chain defined by saturated linear chains with 6 to 8 carbon atoms and X is defined as a hydrogen or iodine atom.

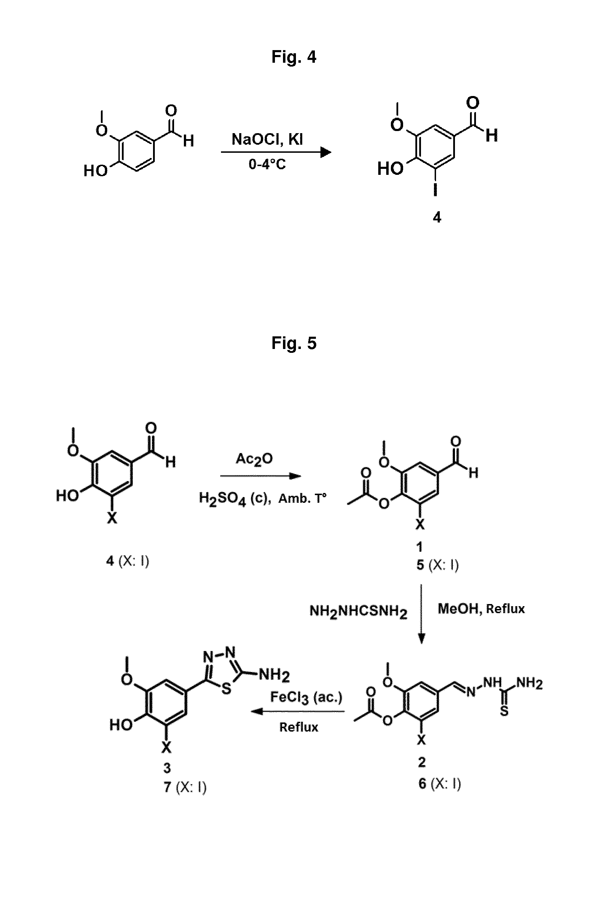

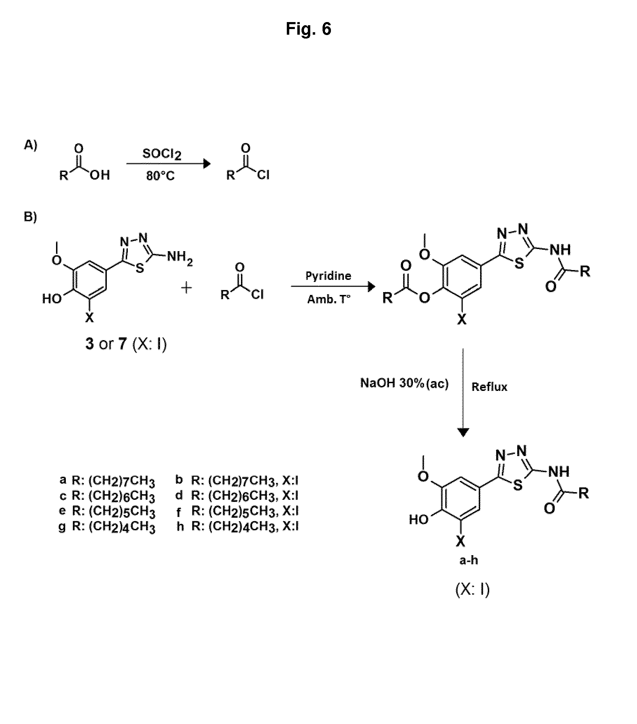

The synthesis method used to synthesize these derivatives begins with the protection by acylation with acetic anhydride of the 4-hydroxyl group of the starting material corresponding to 4-Hydroxy-3-methoxybenzaldehyde or vanillin. The iodinated derivatives in C5 of the vanillyl group are obtained through an iodination reaction with potassium iodide and commercial sodium hypochlorite, as shown in **Figure 4****;** subsequently the hydroxyl group of this product corresponding to 4-Hydroxy-3-iodo-5-methoxybenzaldehyde or 5-lodovanillin was protected with acetic anhydride. The 1, 3, 4-thiadiazole heterocycle was obtained using the oxidative cyclisation of thiosemicarbazone method, shown in **Figure 5****,** where the aldehyde of the respective protected compounds vanillin and 5-lodovanillin were condensed with thiosemicarbazone to obtain the thiosemicarbazone derivatives, from which through oxidative cyclization with ferric chloride in an aqueous medium the derivates of 1, 3, 4-thiadiazole-2-amino with the unprotected hydroxyl group were obtained in the same reaction stage. Subsequently, these products of 1, 3, 4-thiadiazole-2-amino were acylated according to the route shown in **Figure 6** with derivatives of acyl chlorides corresponding to acid chlorides with linear hydrocarbonated chains of 6-9 carbon atoms, and the results of this acylation reaction were products acylated both in the hydroxyl and amino groups, and the final synthesis phase therefore consisted of the basic hydrolysis of the ester formed to obtain the respective compounds described in **Figure 1** with the free hydroxyl group, thus making it possible to obtain the following compounds:
**[a]** N-[5-(4-hydroxy-5-methoxy-3-iodophenyl)-1,3,4-thiadiazole-2-yl]- nonamide,
**[b]** N-[5-(4-hydroxy-3-methoxyphenyl)-1, 3, 4- thiadiazole-2-yl]- nonamide,
**[c]** N-[5-(4-hydroxy-5-methoxy-3-iodophenyl)-1,3,4-thiadiazole-2-yl]-octanamide,
**[d]** N-[5-(4-hydroxy-3-methoxyfenil)-1, 3, 4- thiadiazole-2-yl]-octanamide,
[**e**]N-[5-(4-hydroxy-5-methoxy-3-iodophenyl)-1,3,4-thiadiazole-2-yl]heptanamide,
**[f]** N-[5-(4-hydroxy-3-methoxyfenil)-1, 3, 4- thiadiazole-2-yl]-heptanamide,
**[g]N-[5-(4-hydroxy-5-methoxy-3-iodophenyl)-1,3,4-thiadiazole-2-yl]-hexanamide,** y
**[h]** N-[5-(4-hydroxy-3-methoxyfenil)-1, 3, 4-thiadiazole-2-yl]-hexanamide.

The second aspect of this invention is related to chalcone derivatives or alpha, beta-unsaturated carbonyl derivatives, as described in **Figures 2 and 3****.**

Where the different R's correspond to the different substituent groups:
R1 corresponds to a hydrogen, a methoxyl group or a hydroxyl group;
R2 is a hydrogen or a methoxyl group;
R3 is a hydrogen or a chlorine group;
R4 is a hydrogen or a bromine group; and
R5 is a hydrogen, a methoxyl group or a hydroxyl group;

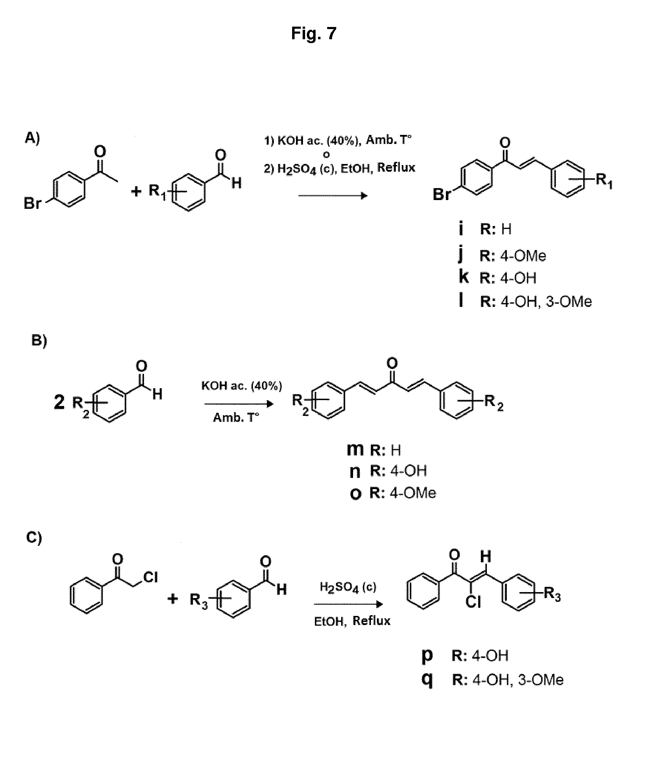

The synthesis method for the compounds described in **Figures 2** and **3** consisted in applying Claisen-Schmidt condensation between different benzaldehydes and acetophenones. The condensation reactions were carried out under basic and acidic catalytic conditions at ambient temperature and under reflux conditions, respectively, as shown in **Figure 7****.** The combination of these different substituents led to the following compounds:
**[i]** *(2E)*-1-(4-bromodiphenyl)-3-phenylprop-2-en-1-one,
**[j]** *(2E)*-1-(4-bromodiphenyl)-3-(4-methoxyphenyl)prop-2-en-1-one,
**[k]** *(2E)*-1-(4-bromodiphenyl)-3-(4-hydroxyphenyl)prop-2-en-1-one,
**[I]** *(2E)*-1-(4-bromodiphenyl)-3-(4-hydroxy-3-methoxyphenyl)prop-2-en-1-one,
**[m]** *(1E, 4E)*-1,5-diphenylpenta-1,4-dien-3-one,
**[n]** *(1E, 4E)*-1,5-(4-hydroxydiphenyl)penta-1,4-dien-3-one,
**[o]** *(1E, 4E)*-1,5-(4-metoxydiphenyl)penta-1,4-dien-3-one,
**[p]** *(2E)*-2-chlorine-3-(4-hydroxyphenyl)-1-phenylprop-2-en-1-one,
**[q]** *(2E)*-2-chlorine-3-(4-hydroxy-3-methoxyphenyl)-1-phenylprop-2-en-1-one.

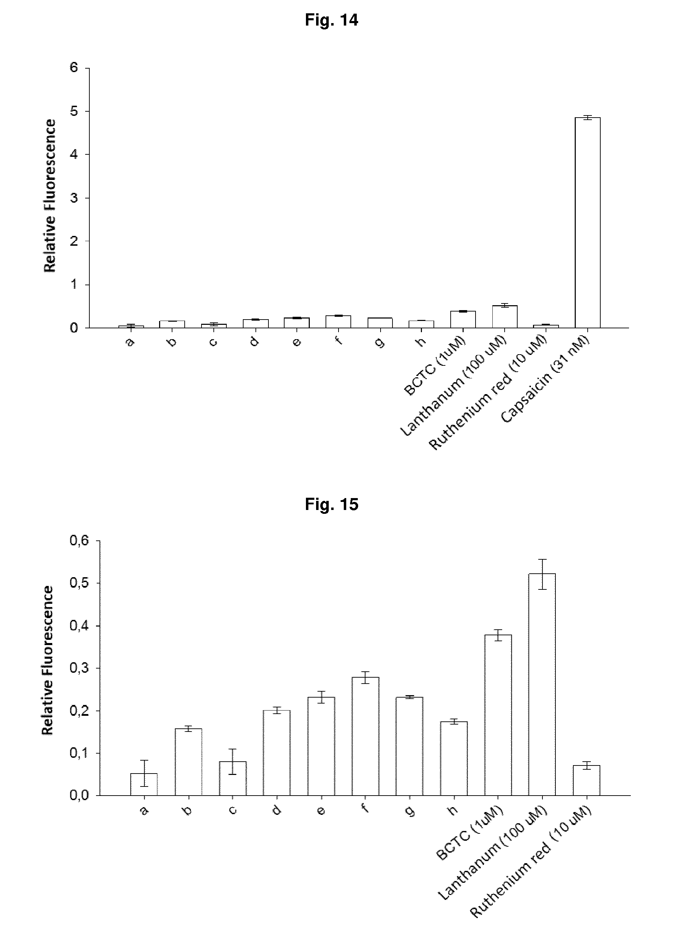

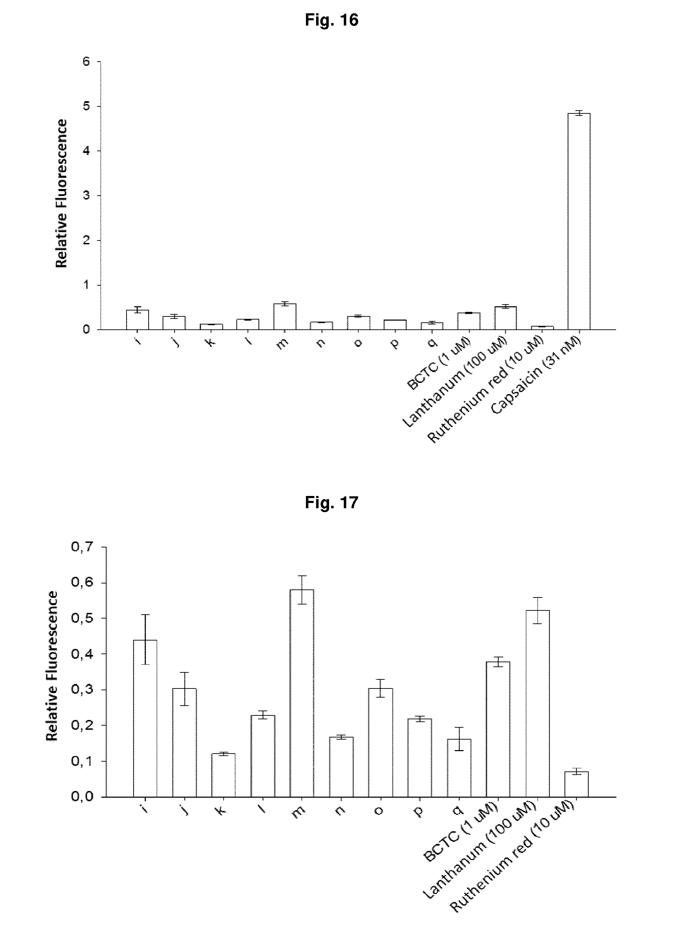

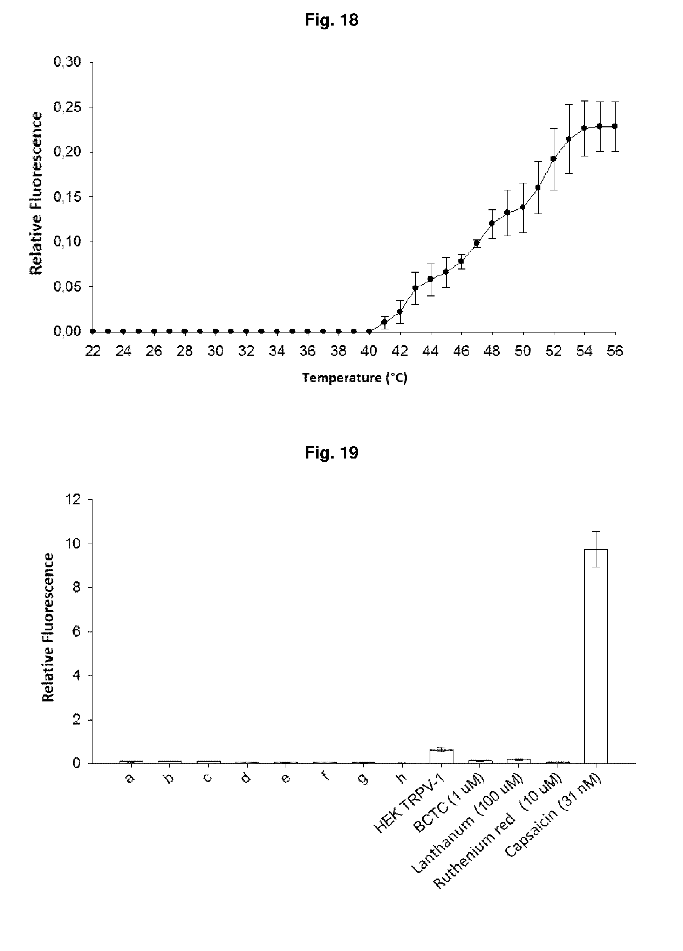

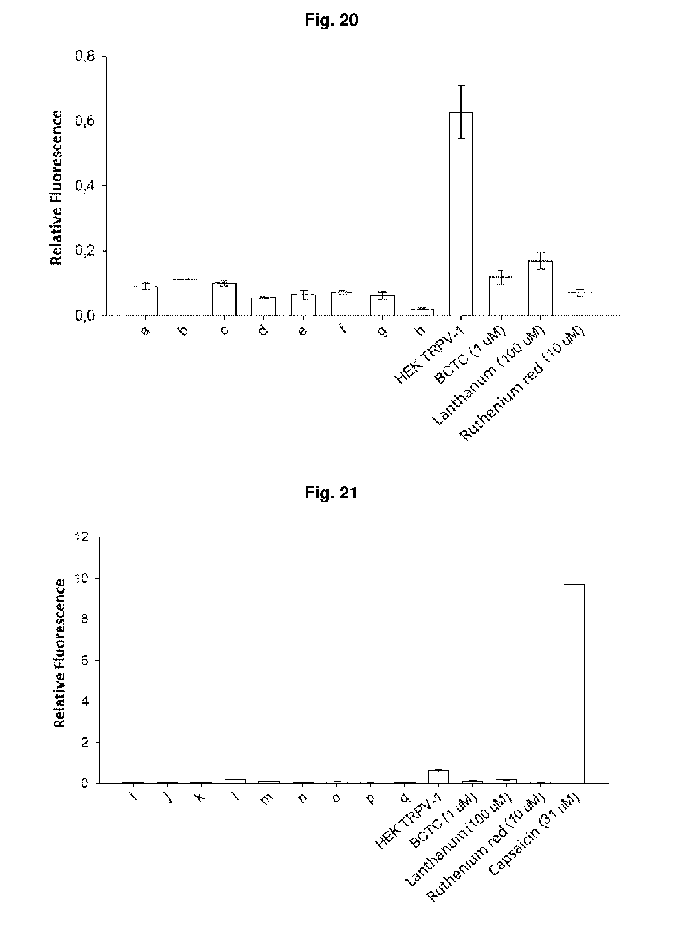

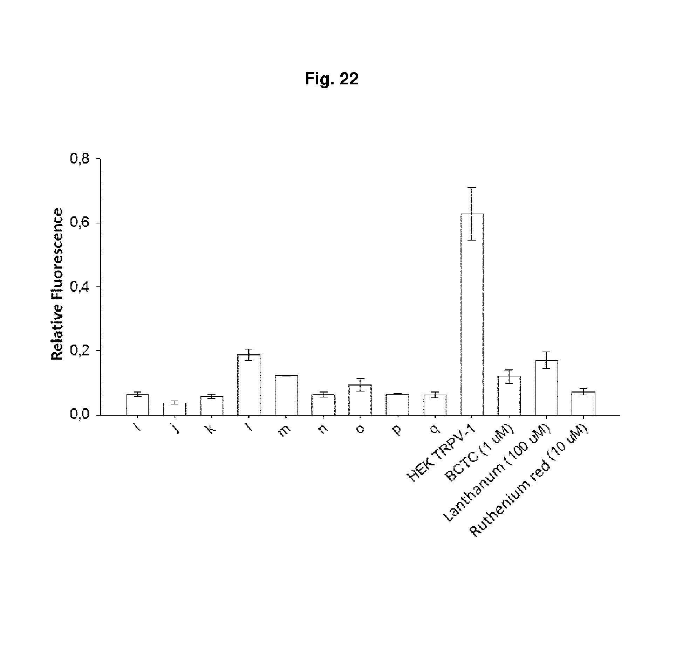

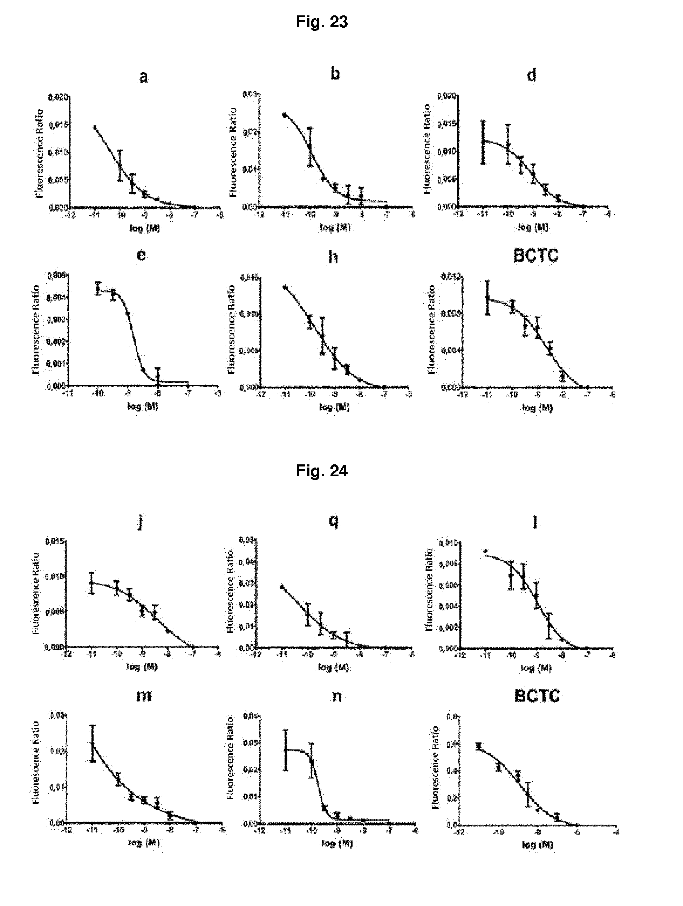

These compounds present themselves as an alternative for treatment of diseases involving a overactivation of the TRPV-1 receptors. In vitro tests done using rat HEK-293T cell cultures with overexpressed TRPV-1, based on fluorometric measurements of inflow of induced calcium, after the activation of the TPRV-1 receptor by capsaicin, indicated that the compounds **[a-h]** and **[i-q]** inhibit the inflow of calcium, observed by a reduction in cellular fluorescence, exhibiting strong antagonistic activity for the activation mode of the TRPV-1 receptor by capsaicin **(****Figures 14-17****).** These compounds, **[a-h]** y **[i-q],** also show antagonistic activity on the temperature activation mode of the TRPV-1 receptor **(****Figures 19-21****),** this activity being represented by the average of relative fluorescence obtained at 54°C in two independent assays, the temperature at which the receptor TRPV-1 presents its maximum activation. From the respective dose-response curves for the compounds with antagonistic activity presented in **Figures 22** and **23****,** the half maximal inhibitory concentration (IC₅₀) was determined as presented in **Tables 7** and **8,** these values indicating antagonistic activity in the nanomolar order, with IC₅₀ ranging from 0.4-1.2 nM, demonstrating that these compounds are potent antagonists of the TRPV-1 receptor, with an activity superior to that shown by many competitive antagonists and by antagonists derived from vanilloid compounds, which are associated with a different mode of binding on the orthosteric site of the TRPV-1 receptor and the establishment of hydrophobic interactions on an undescribed binding site that contributes to the increase in antagonistic activity, based on the structure-activity analysis and conformational aspects of the synthesized compounds related to the planarity and conformational restriction imposed by the 1, 3, 4-thiadiazole heterocycle and the alpha, beta unsaturated carbonyl system, present in compounds **[a-h]** and **[i-q],** respectively.

The compounds described here, are characterized mainly for exhibiting antagonistic activity on TRPV-1 receptors with an increased conformational restriction, which contributes to the increase of ligand-receptor binding energy and affects the greater antagonistic activity, and also for acquiring a different conformation within the pharmacophore regions, to that associated previously with antagonistic activity. Thus, in the derivatives of 1, 3, 4-thiadiazole alkylamides, the conformational restriction is regulated through the isosteric replacement of the amide group present in capsaicin analogues, by the 1, 3, 4-thiadiazole heterocycle, which allows the synthesized compounds to acquire a coplanar conformation between the donor-acceptor region of the hydrogen bonds of the vanillyl group and the polar region of the 1, 3, 4-thiadiazole group, while the incorporation of an amide group as a spacer allows for a double conformation between the donor-acceptor region of the hydrogen bonds (vanillyl group) and the hydrophobic region made up of aliphatic linear chains. This permits the obtention of iodinated and non-iodinated vanilloid analogs with a potent antagonistic activity, the effect of the latter being the opposite to the effect shown by vanilloid compounds with a non-iodinated vanillyl group, which usually have agonistic activity with the exception of capsazepine, a non-iodinated vanilloid derivative and the first identified competitive antagonist of capsaicin; however, it is of low potency (IC₅₀: 56.2 nM), metabolically unstable and has a low selectivity for the TRPV-1 receptor (23.45) unlike the derivatives of 1, 3, 4-thiadiazole alkylamides, which have a high conformational restriction favoring their selectivity for TRPV-1 receptors. In this way the conformational restriction imposed by the 1, 3, 4-thiadiazole heterocycle and the amide group, allow for the stabilization of the conformational state of the TRPV-1 receptor responsible for its inactivation, which explains the antagonism exhibited by both non iodinated and iodinated derivatives in the vanilloid group that are associated with a different mode of union than that used by antagonistic vanilloid compounds on the orthosteric binding site of the receptor.

With derivatives of chalcones or alpha, beta unsaturated carbonyls, the conformational restriction is regulated through a double unsaturated bond and the elimination of spacer groups commonly found in compounds with activity on receptors TRPV-1, such as amino, oxo or methylene groups, which have been shown to influence agonistic and antagonistic activity; thus it has been revealed that with capsaicin analogues with agonistic activity, the substitution of the methylene group between the vanillyl group (donor-acceptor region of hydrogen bonds) and amide (polar region) by an ethylene or carbonyl group notably reduces the agonistic activity of the compounds (47), while the absence of this spacer region in capsaicin and thiourea analogs eliminates both agonistic and antagonistic activity respectively (47,48,49). Hence the structural changes obtained through the synthesis of chalcone derivatives allow for the obtention of potent antagonists of the TRPV-1 receptor with nanomolar activity, which in addition to being associated with a greater conformational restriction is related to the anti-coplanar conformation between the donor-acceptor region of the hydrogen bonds and the hydrophobic region formed by the aryl groups. The aforementioned changes also permit the obtention of molecules with a low molecular weight which facilitate the pharmacological development of orally administered TRPV-1 receptor antagonists to increase bioavailability.

### EXAMPLES OF APPLICATION

### EXAMPLE 1: Synthesis of the compounds

### General Procedures

The synthesis reactions for each of the proposed compounds were carried out in solvents without prior treatment, except for the acylation reactions, in which the pyridine catalyst used was previously dried with solid KOH. The course of the reactions was evaluated with fine layer chromatography using plates of silica gel and 60% ethyl acetate/petrol as a mobile phase. The plates were revealed with ultraviolet light, and solutions of vanillin/sulfuric acid/methanol, 30% sulfuric acid/methanol and Folin reagent. The products were purified through crystallization or column chromatography, and the respective analysis of spectroscopic characterization were done on an infrared spectrometer, using a nuclear magnetic resonance spectrometer for the analysis of RMN-¹H and RMN-¹³C.

The following are the first seven compounds, corresponding to the reaction intermediates described in **Figures 4** and **5**

### 4-Formyl-2-methoxyphenyl acetate (1).

10g (0.66 moles) of 4-Hydroxy-3-methoxybenzaldehyde and 40ml of acetic anhydride are added to a 100ml Erlenmeyer matrix. Then 3 drops of concentrated sulfuric acid are added during stirring. The reaction mixture is left under stirring at room temperature for four hours, after which 40ml of distilled water is added to the reaction medium. The resulting solid is filtered and crystallized in methanol, obtaining 9.13g (0.047 moles) of light pink-orange crystals with a yield of 71.27%.

### 4-(carbamothioylhydrazinylidene) methyl-2-methoxyphenyl acetate (2).

In a 250ml round bottomed flask, 8g (0.041 moles) of 4-formyl-2 methoxyphenyl and 3.73g (0.041 moles) of thiosemicarbazide are added to 50ml of methanol, the reaction medium is acidified with glacial acetic acid until it reaches a pH of 4-5 and then the reaction mixture is refluxed for 6 hours under stirring. Afterwards, it is cooled in an ice-water bath resulting in the formation of a yellow-white precipitate, which is filtered and crystallized in methanol, obtaining 9.37g (0.036 moles) of the crystals with a yield of 89.2%.

### 2-amino-5-(4-hydroxy-3- methoxyphenyl)-1, 3, 4 thiadiazole (3).

5g (0.0195 moles) of thiosemicarbazone, 20 ml (9.48g; 0.059 moles) of an aqueous solution of ferric chloride and 30ml of methanol are added to a 250ml round bottomed flask. The reaction mixture is refluxed under stirring for a period of 1-2 hours. Subsequently, the reaction mixture is filtered with activated carbon while hot, and then 30ml of an aqueous solution of citric acid (11.52g; 0.06 moles) and sodium citrate (6.42g; 0.03 moles) are added to the filtrate, which is refluxed for 1 hour under stirring. The reaction mixture is neutralized with 10% ammoniac at a pH of 4-5 and then cooled in an ice-water bath, resulting in the formation of a yellow precipitate which is crystallized various times in a mixture of ethanol/methanol, obtaining 2.61 g (0.0116 moles) of the product as a yellow powder with a yield of 60%.

### 4-hydroxy-5-methoxy-3- iodobenzaldehyde (4).

In an Erlenmayer flask, 10g (0.065 moles) of 4-hydroxy-3-methoxybenzaldehyde and 9.73g (0.065 moles) of potassium iodide are dissolved in 20ml of methanol. The solution is cooled in an ice-water bath for 15 minutes. Using an addition funnel and under stirring in the ice-water bath, 74ml (0.065 moles) of commercial NaOCl (4.7% v/v) are added, drop by drop, to the reaction mixture. Once all of the NaOCl has been added, the reaction is stirred for another 60 minutes in the ice-water bath. Subsequently, 20ml of a 10% sodium thiosulfate solution (%p/p) are added and the mixture is stirred for 15 minutes at room temperature. The reaction mixture is then acidified with 37% HCL until reaching a pH of 3-4, and the resulting precipitate is filtered then washed with ice water. The solid is left to dry and crystallize in isopropyl alcohol, obtaining 13.54g (0.048 moles) of the product as light yellow crystals with a yield of 75.22%.

### 4-formyl-6-methoxy-2-iodophenyl acetate (5).

In an Erlenmeyer flask are placed 4 g (0.0144 moles) of 4-hydroxy-5-methoxy-3-iodobenzaldehyde and 20ml of acetic anhydride. Under stirring, 3 drops of H₂SO₄ (c) are added. The reaction mixture is left stirring at room temperature for 6 hours, after which 30ml of distilled water are added, forming a precipitate that is then filtered and washed several times with distilled water. Subsequently the solid is crystallized in methanol, obtaining 4.57g (0.0142 moles) of the product as light brown crystals with a 99.13% yield.

### 4-(carbamothioylhydrazinylidene) methyl-6-methoxy-2-iodophenyl acetate (6).

In a round bottomed flask, 4g (0.012 moles) of 4-formyl-6-methoxy-2-iodophenyl acetate and 1.09g (0.012 moles) of thiosemicarbazide are added to 50 ml of methanol, the reaction medium is acidified with glacial acetic acid until a pH of 4-5 is reached and the reaction mixture is refluxed for six hours under stirring. It is then left to cool in an ice-water bath, resulting in the formation of a yellow-white precipitate, which is filtered and crystallized in methanol, obtaining 4.03g (0.01 moles) of yellow-white crystals in a yield of 85.38%.

### 2-amino-5-(4-hydroxy-5-methoxy-3-iodophenyl)-1, 3, 4-thiadiazole (7).

In a round bottomed flask, 4g (0.016 moles) of thiosemicarbazone, and 5.2g (0.032 moles) of FeCl₃ are dissolved in 10ml of distilled water and 20ml of methanol. The reaction mixture is refluxed for 2 hours, after which 20ml of a citric acid (6.15g; 0.032 moles) and sodium citrate (3.43g; 0.016 moles) solution are added, the resulting mixture being stirred under reflux for 1 hour. It is then heated in activated carbon and filtered while hot. The filtrate is concentrated to half its volume and is left to cool in an ice-water bath, resulting in the formation of a light brown solid which is crystallized several times with water and methanol obtaining 1.66g (0.0047 moles) with a yield of 44.86%.

The synthesis of compounds **[a-h]** and **[i-q]** described in **Figures 6** and **7** are presented in the following:

### N-[5-(4-hydroxy-3-methoxyphenyl)-1, 3, 4-thiadiazole-2-yl] nonanamide (a).

2ml (0.011 moles) of nonanoic acid and 0.6ml (0.0083 moles) of thionyl chloride are added to a previously dried round bottomed flask, the mixture then being stirred and refluxed for 16 hours at 80°C. The mixture is left to cool at room temperature and under vigorous stirring 0.2g (0.000895 moles) of 2-amino-5-(4-hydroxy-3-methoxyphenyl)-1, 3, 4 thiadiazole are quickly added, followed by 2ml of pyridine. The reaction mixture is left under stirring at room temperature for 2 hours, obtaining the diacylated product, of which 0.3g (0.0006 moles) are placed in a previously dried round bottomed flask, followed by 2ml of a 30% NaOH solution in 10ml of methanol, refluxed for 3 hours under stirring. Once the reaction has reached room temperature it is acidified to a pH of 4 with 37% HCL, thus obtaining a white solid that is purified with column chromatography, using 20% hexane/ethyl acetate as a mobile phase. Lastly, the product is crystallized in methanol water, obtaining 0.185g of a white solid with a yield of 84.86%.

### N-[5-(4-hydroxy-5-methoxy-3-iodophenyl)-1,3,4-thiadiazole-2-yl] nonanamide (b).

In a previously dried round bottomed flask are added 1ml (0.011 moles) of nonanoic acid and 0.6ml (0.0083 moles) of thionyl chloride. The mixture is stirred and refluxed for 16 hours at 80°C, then left to cool at room temperature, after which, and under vigorous stirring, 0.2g (0.000573 moles) of 5-(4-hydroxy-5-methoxy-3-iodophenyl)-1, 3, 4 thiadiazole-2-amino are quickly added, followed by 2ml of pyridine. The reaction mixture is left under stirring at room temperature for 2 hours, after which the reaction is partitioned between an acid aqueous solution and dichloromethane. The organic phase is extracted and washed with a saturated NaCl solution, then concentrated, and the residue obtained is hydrolyzed in a 30% NaOH solution in methanol for 3 hours under reflux and stirring. Once the reaction reaches room temperature it is acidified to a pH of 4 with 37% HCL, hereby obtaining a brown solid, which is purified with column chromatography using 20% hexane/ethyl acetate as the mobile phase. Finally the obtained product is crystallized in methanol water, obtaining 0.064g of a brown solid with a yield of 22.85%.

### N-[5-(4-hydroxy-3-methoxyphenyl)-1, 3, 4-thiadiazole-2-yl] octanamide (c).

1 ml (0.013 moles) of octanoic acid and 0.8ml (0.011 moles) of thionyl chloride are added to a previously dried round bottomed flask. The mixture is stirred and refluxed for 16 hours at 80°C, then left to cool at room temperature. Under vigorous stirring 0.2g (0.000895 moles) of 5-(4-hydroxy-3-methoxyphenyl)-1, 3, 4 thiadiazole-2-amino are quickly added, followed by 2ml of pyridine. The reaction mixture is left stirring at room temperature for 2 hours, after which the reaction is partitioned between an acid aqueous solution and dichloromethane. The organic phase is extracted and washed with a saturated NaCL solution, then concentrated and the residue obtained is hydrolyzed in a 30% NaOH solution in methanol for 3 hours under reflux and stirring. Once the reaction reaches room temperature it is acidified to a pH of 4 with 37% HCL, thus obtaining a white solid which is purified by column chromatography using 20% hexane/ethyl acetate as a mobile phase. Lastly, the obtained product is crystallized in methanol water, obtaining 0.173g of a white solid with a yield of 55.27%.

### N-[5-(4-hydroxy-5-methoxy-3-iodophenyl)-1,3,4-thiadiazole-2-yl] octanamide (d).

To a previously dried round bottomed flask are added 1ml (0.013 moles) of octanoic acid and 0.8ml (0.011 moles) of thionyl chloride. The mixture is stirred and refluxed for 16 hours at 80°C, then left to cool at room temperature. Under vigorous stirring 0.2g (0.000573 moles) of 5-(4-hydroxy-5-methoxy-3-iodophenyl)-1, 3, 4 thiadiazole-2-amino are quickly added, followed by 2ml of pyridine. The reaction mixture is left stirring at room temperature for 2 hours, after which the reaction is partitioned between an acid aqueous solution and dichloromethane. The organic phase is extracted and washed with a saturated NaCl solution, then concentrated and the residue obtained is hydrolyzed in a 30% NaOH solution in methanol for 3 hours under reflux and stirring. Once the reaction reaches room temperature it is acidified to a pH of 4 with 37% HCl, thus obtaining a brown solid that is purified by column chromatography, using 20% hexane/ethyl acetate as the mobile phase. Finally the product is crystallized in methanol water, obtaining 0.091 g of a brown solid with a yield of 33.46%.

### N-[5-(4-hydroxy-3-methoxyphenyl)-1, 3, 4-thiadiazole-2-yl] heptanamide (e).

1 ml (0.014 moles) of heptanoic acid and 0.9ml (0.012 moles) of thionyl chloride are added to a previously dried round bottomed flask. The mixture is stirred and refluxed for 16 hours at 80°C, then left to cool at room temperature. Under vigorous stirring 0.2g (0.000895 moles) of 5-(4-hydroxy-3-methoxyphenyl)-1, 3, 4 thiadiazole-2-amino are quickly added, followed by 2ml of pyridine. The reaction mixture is left stirring at room temperature for 2 hours and then partitioned between an acid aqueous solution and dichloromethane. The organic phase is extracted and washed with a saturated NaCl solution, then concentrated, and the residue obtained is hydrolyzed in a 30% NaOH solution in methanol for 3 hours under reflux and stirring. Once the reaction reaches room temperature it is acidified to a pH of 4 with 37% HCl, thus obtaining a white solid that is purified with column chromatography, using 20% hexane/ethyl acetate as the mobile phase. Lastly the product is crystallized in methanol water, obtaining 0.167g of a white solid with a yield of 55.48%.

### N-[5-(4-hydroxy-5-methoxy-3-iodophenyl)-1,3,4-thiadiazole-2-yl] heptanamide (f).

1 ml (0.014 moles) of heptanoic acid and 0.9ml (0.012 moles) of thionyl chloride are added to a previously dried round bottomed flask. The mixture is stirred and refluxed for 16 hours at 80°C, then left to cool at room temperature. Under vigorous stirring 0.2g (0.000573 moles) of 2-amino-5-(4-hydroxy-5-methoxy-3-iodophenyl)-1, 3, 4 thiadiazole are quickly added, followed by 2 ml of pyridine. The reaction mixture is left stirring at room temperature for 2 hours, after which the reaction is partitioned between an acid aqueous solution and dichloromethane. The organic phase is extracted and washed with a saturated NaCl solution, then concentrated, and the resulting residue is hydrolyzed in a 30% NaOH solution in methanol for 3 hours under reflux and stirring. Once the reaction reaches room temperature it is acidified to a pH of 4 with 37% HCl, thus obtaining a brown solid that is purified with column chromatography using 20% hexane/ethyl acetate as the mobile phase. Finally the product is crystallized in methanol water, obtaining 0.078g of a brown solid with a yield of 29.55%.

### N-[5-(4-hydroxy-3-methoxyphenyl)-1, 3, 4-thiadiazole-2-yl] hexanamide (g).

1 ml (0.016 moles) of hexanoic acid and 0.9ml (0.012 moles) of thionyl chloride are added to a previously dried round bottomed flask. The mixture is stirred and refluxed for 16 hours at 80°C, then left to cool at room temperature. Under vigorous stirring 0.2g (0.000895 moles) of 2-amino-5-(4-hydroxy-3-methoxyphenyl)-1, 3, 4 thiadiazole are quickly added, followed by 2 ml of pyridine. The reaction mixture is left stirring at room temperature for 2 hours, after which the reaction is partitioned between an acid aqueous solution and dichloromethane. The organic phase is extracted and washed with a saturated NaCl solution, then concentrated, and the resulting residue is hydrolyzed in a 30% NaOH solution in methanol for 3 hours under reflux and stirring. Once the reaction reaches room temperature it is acidified to a pH of 4 with 37% HCl, thus obtaining a white solid that is purified with column chromatography, using 20% hexane/ethyl acetate as the mobile phase. Lastly the product is crystallized in methanol water, obtaining 0.0118g of a white solid with a yield of 41.11 %

### N-[5-(4-hydroxy-5-methoxy-3-iodophenyl)-1,3,4-thiadiazole-2-yl] hexanamide (h).

1ml (0.016 moles) of hexanoic acid and 0.9ml (0.012 moles) of thionyl chloride are added to a previously dried round bottomed flask. The mixture is stirred and refluxed for 16 hours at 80°C then left to cool at room temperature. Under vigorous stirring 0.2g (0.000573 moles) of 2-amino-5-(4-hydroxy-5-methoxy-3-iodophenyl)-1, 3, 4 thiadiazole are quickly added, followed by 2ml of pyridine. The reaction mixture is left stirring at room temperature for 2 hours after which the reaction is partitioned between an acid aqueous solution and dichloromethane. The organic phase is extracted and washed with a saturated NaCl solution then concentrated, and the obtained residue is hydrolyzed in a 30% NaOH solution in methanol for 3 hours under reflux and stirring. Once the reaction reaches room temperature it is acidified to a pH of 4 with 37% HCl, thus obtaining a brown solid that is purified with column chromatography, using 20% hexane/ethyl acetate as the mobile phase. Finally the product is crystallized in methanol water obtaining 0.058g of a brown solid with a yield of 22.66%.

### (2E)-1-(4-bromophenyl)-3-phenylprop-2-en-1-one (i)

In an Erlenmayer flask, 2g (0.01 moles) of 4-bromoacetophenone and 1.02ml (1.06g; 0.01 moles) of benzaldehyde are dissolved in 30ml of methanol. Under stirring on a stirring plate, 2ml of an aqueous solution of 50% KOH are added drop by drop, and the reaction mixture is stirred for 18 hours at room temperature. The mixture is then neutralized to pH7 with 37% HCl and left to cool in an ice bath for 24 hours, resulting in the formation of a light yellow precipitate, which is filtered and washed with copious amounts of distilled water. The solid is recrystallized in methanol, obtaining 2.42g of the product with an 84.32% yield.

### (2E)-1-(4-bromophenyl)-3-(4-methoxyphenyl) prop-2-en-1-one (j)

2g (0.01 moles) of 4-bromoacetophenone and 1.22ml (1.36g; 0.01 moles) of anisaldehyde are dissolved in an Erlenmayer flask. Under stirring on a stirring plate, 2ml of an aqueous solution of 50% KOH are added drop by drop and the reaction mixture is stirred for 18 hours at room temperature. The mixture is then neutralized to a pH of 7 with 37% HCl and left to cool in an ice bath for 24 hours, resulting in the formation of a light yellow precipitate, which is filtered and washed with copious amounts of distilled water. The solid is recrystallized in methanol, obtaining 2.48g (0.007 moles) of the product with a 78.2% yield.

### (2E)-1-(4-Bromophenyl)-3-(4-hydroxyphenyl) prop-2-en-1-one (k)

In a round bottomed flask, 2g (0.01 moles) of 4-bromoacetophenone and 1.22g (0.01 moles) of 4-hydroxybenzaldehyde are dissolved in 20ml of absolute ethanol. 1 ml of H₂SO₄ (c) is added and the mixture is stirred under reflux at 60°C for 18 hours. It is then neutralized to a pH of 7 with NaOH (c) and the organic phase is extracted with dichloromethane and is concentrated. The residue is purified with column chromatography using 30% hexane/ethyl acetate as the mobile phase, obtaining 0.875g (0.003 moles) of the product as a yellow solid with a 28.9% yield.

### (2E)-1-(4-bromophenyl)-3-(4-hydroxy-3-methoxyphenyl) prop-2-en-1-one (I)

In a round bottomed flask, 2.58g (0.01 moles) of 4-bromoacetophenone and 2g (0.01 moles) of 4-hydroxy-3-methoxybenzaldehyde are dissolved in 20ml of absolute ethanol. 1 ml of H₂SO₄ (c) is added and the mixture is refluxed under stirring at 60°C for 18 hours. It is then neutralized to a pH of 7 using NaOH (c) and the organic phase is extracted with dichloromethane and is concentrated. The residue is purified with column chromatography using 30% hexane/ethyl acetate as the mobile phase, obtaining 0.789g (0.002 moles) of the pure product as an orange solid with an 18.2% yield.

### (1E, 4E)-1,5-diphenyl penta-1,4-dien-3-one (m)

In an Erlenmayer flask, 2g (0.019 moles) of benzaldehyde and 0.69ml (0.55g; 0.0095 moles) of acetone are dissolved in 10ml of methanol. Under stirring, and drop by drop, 2ml of a 40% solution of KOH are added. Once this addition process is finished, the reaction mixture is left stirring for 30 additional minutes at room temperature. A yellow precipitate is formed, which is then filtered and crystallized in methanol. Thus 4.23g (0.018 moles) of yellow crystals are obtained with a 95.05% yield.

### (1E, 4E)-1,5-(4-hydroxyphenyl) penta-1,4-dien-3-one (n)

In an Erlenmeyer flask, 2g (0.016 moles) of 4-hidroxybenzaldehyde and 0.58ml (0.46g; 0.008 moles) of acetone are dissolved in 10ml of methanol. Under stirring and drop by drop, 2ml of a 40% KOH solution are added, after which the reaction mixture is left stirring for 30 minutes at room temperature. The resulting reaction is acidified to a pH of 4 with 37% HCl and the organic phase is extracted with dichloromethane and is concentrated. The residue is crystallized three times in methanol/water, obtaining 0.324g (0.001 moles) of product as light orange crystals with a 7.6% yield.

### (1E, 4E)-1,5-(4-methoxyphenyl) penta-1,4-dien-3-one (o)

In an Erlenmeyer flask, 2g (0.015 moles) of 4-methoxybenzaldehyde and 0.53ml (0.42g; 0.0073 moles) of acetone are dissolved in 10ml of methanol. Under stirring, 2ml of a 40% KOH solution are added drop by drop. Once the solution has been added, the mixture is left stirring for 30 minutes at room temperature. A yellow precipitate is formed, which is filtered and crystallized in methanol, obtaining 2.52g (0.00086 moles) of yellow crystals with a 58.74% yield.

### (2E)chloro-3-(4-hydroxyphenyl)-1-phenylprop-2-en-1-one. (p)

In a round bottomed flask, 2g (0.01 moles) of phenacyl chloride and 1.22g (0.01 moles) of 4-hydroxybenzaldehyde are dissolved in 20ml of absolute ethanol. 1 ml of H₂SO₄ (c) is added and under stirring the mixture is refluxed at 60°C for 18 hours. It is then neutralized to a pH of 7 with NaOH (c) and the organic phase is extracted with dichloromethane and is concentrated. The residue is purified with column chromatography, using 30% hexane/ethyl acetate as the mobile phase, obtaining 0.834g (0.004 moles) of the product as a light orange solid with a 37.23% yield.

### (2E)-chloro-3-(4-hydroxy-3-methoxyphenyl)-1-phenyl prop-2-en-1-one (q).

In a round bottomed flask, 1.95g (0.01 moles) of phenacyl chloride and 2g (0.01 moles) of 4-hydroxy-3-methoxybenzaldehyde are dissolved in 20 ml of absolute ethanol. 1ml of H₂SO₄ (c) is added and under stirring the reaction mixture is refluxed at 60°C for 18 hours. It is then neutralized to a pH of 7 with NaOH (c) and the organic phase is extracted with dichloromethane and is concentrated. The residue is purified with column chromatography using 30% hexane/ethyl acetate as the mobile phase, obtaining 0.796g (0.003 moles) of the product as an orange solid with a 31.33% yield.

### EXAMPLE 2: Characterization of the compounds

### Retention Factor:

The retention factor for the purified vanilloid and chalcone derivative compounds is determined by column chromatography or crystallization. The respective values are presented in **Tables 1** and **2.**

**Table 1: Retention Factor (Rf) of 1, 3, 4-thiadiazole alkylamide derivatives:**

| Mobile phase | **(a)** | **(b)** | **(c)** | **(d)** | **(e)** | **(f)** | **(g)** | **(h)** |
|---|---|---|---|---|---|---|---|---|
| 60% ethyl acetate /petrol | 0,3 | 0,3 | 0,27 | 0,29 | 0,26 | 0,3 | 0,2 | 0,2 |

**Table 2: Retention Factor (Rf) of chalcone derivatives:**

| Mobile phase | **(i)** | **(j)** | **(k)** | **(l)** | **(m)** | **(n)** | **(o)** | **(p)** | **(q)** |
|---|---|---|---|---|---|---|---|---|---|
| 30% ethyl acetate/ hexane | 0,86 | 0,74 | 0,50 | 0,44 | 0,8 | 0,33 | 0,36 | 0,48 | 0,51 |

### Infrared Spectra:

The characterization with infrared spectroscopy (IR) was carried out through the identification of the respective absorption frequencies of each functional group. **Table 3** presents the characterization of the vanilloid derivative compounds of 1, 3, 4-thiadiazole alkylamides using IR spectroscopy.

**Table 3: Absorption frequencies (KBr) v cm⁻¹:**

| | N-H amide | O-H | C-H sp³ | C-H sp² aromatic | C=O | C=C aromatic |
|---|---|---|---|---|---|---|
| **(a)** | 3538,2 | 3398 | 2920,21-2730,72 | 3155,60 | 1680,21 | 1573, 98;1533,04; 1466,44 |
| **(b)** | 3536,22 | 3334,22 | 2924,58-2853,71 | 3161,33 | 1694,22 | 1560,03; 1502,41; 1463,92 |
| **(c)** | 3308,01 | 3308,01 | 2946,86-2861,81 | 3154,41 | 1672,79 | 1563,81; 1522,28;1456,18 |
| **(d)** | 3386,16 | 3386,16 | 2929,56-2864,87 | 3158,92 | 1695,60 | 1560,42;1503,70;1463,51 |
| **(e)** | 3536,01 | 3212,54 | 2930,79-2775,56 | 3091,83 | 1676,99 | 1565,76; 1519,69;1460 |
| **(f)** | 3400,52 | 3400,52 | 2929,09-2863,98 | 3163,52 | 1696,07 | 1559,19; 1498,60;1461,47 |
| **(g)** | 3402,67 | 3402,67 | 2997,19-2742,07 | 3162,49 | 1679,61 | 1566,99; 1523,72;1457,78 |
| **(h)** | 3404,63 | 3404,63 | 2927,67-2735,99 | 3164,74 | 1699,04 | 1562,54;1497,57;1462,09 |

The absorption frequencies of the chalcone derivative compounds or alpha, beta unsaturated carbonyl derivatives **[i-q],** are presented in **Table 4.**

**Table 4: Absorption frequencies (KBr) v cm⁻¹:**

| | O-H | C-H sp³ | C=O | C=C aromatic | C=C | C-O-C asymmetri c | C-O-C symmetri c |
|---|---|---|---|---|---|---|---|
| **(i)** | | | 1657,24 | 1578,96 | 1602,33 | | |
| | | | | 1440,16 | | | |
| **(j)** | | | 1654,24 | 1508,28 | 1591,49 | 1254,60 | 1030,35 |
| | | | | 1457,82 | | | |
| **(k)** | 3264,75 | | 1644,90 | 1511,87 | 1579,33 | | |
| **(l)** | 3362,78 | | 1647,20 | 1514,85 | 1578,01 | 1274,81 | 1031,25 |
| **(m)** | | | 1628,82 | 1510,96,1433,3 2 | 1597,69 | | |
| **(o)** | | 2959,76 | 1628,05 | 1508,11 | 1597,48 | 1244,03 | 1028,35 |
| | | 2854,39 | | 1416,72 | | | |
| **(n)** | 3423,31 | | 1649,02 | 1492,82,1445,7 5 | 1593,06 | | |
| **(p)** | 3298,05 | | 1642,99 | 1505,81 | 1570,60 | | |
| | | | | 1445,37 | | | |
| **(q)** | 3336,48 | | 1632,47 | 1510,32 | 1568,06 | 1251,18 | 1028,47 |
| | | | | 1463,60 | | | |

### Nuclear Magnetic Resonance

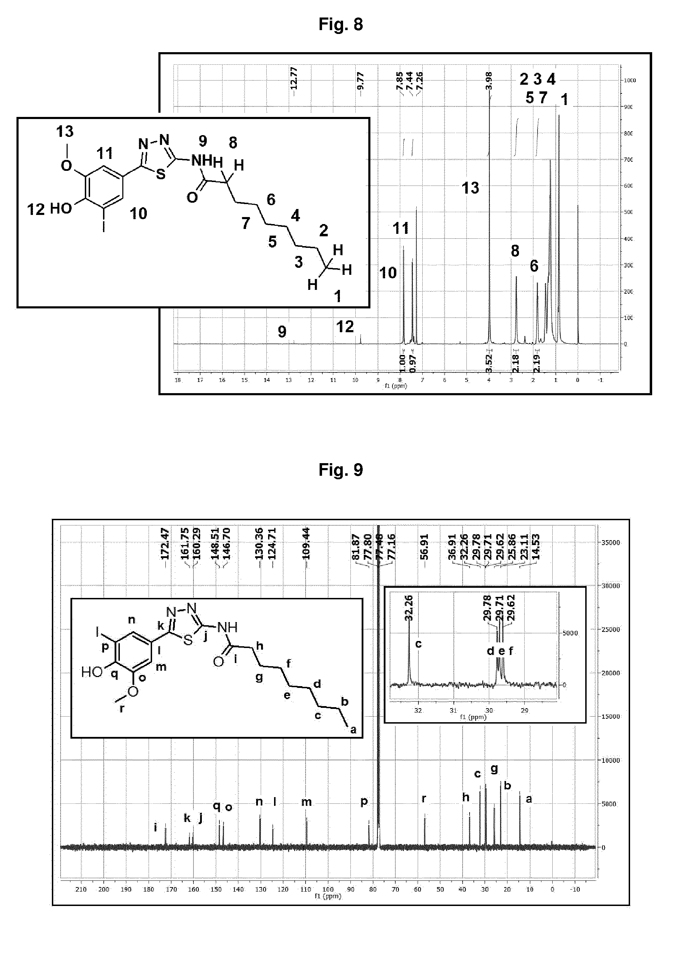

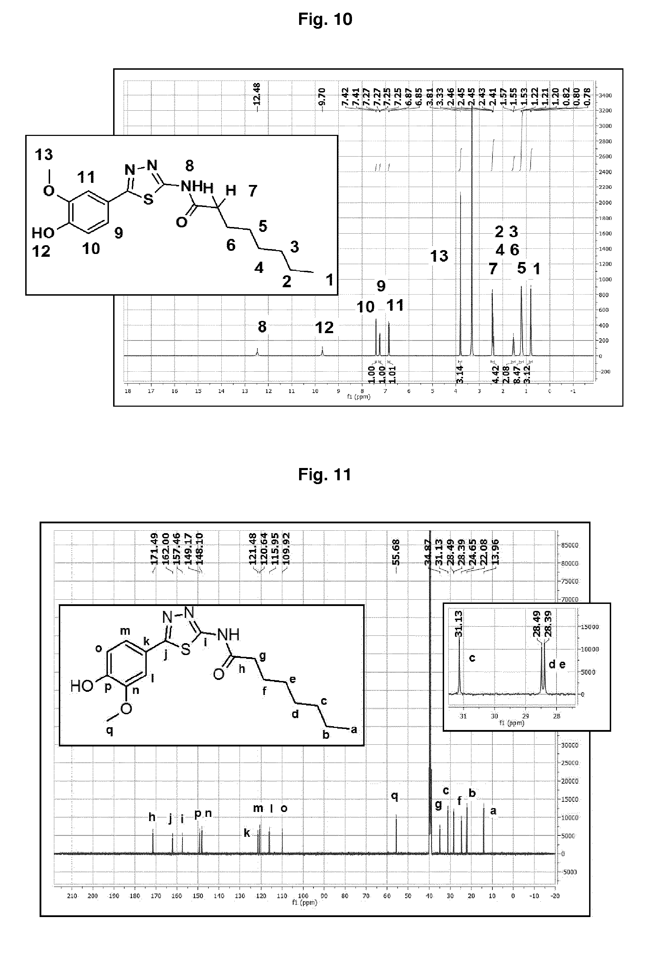

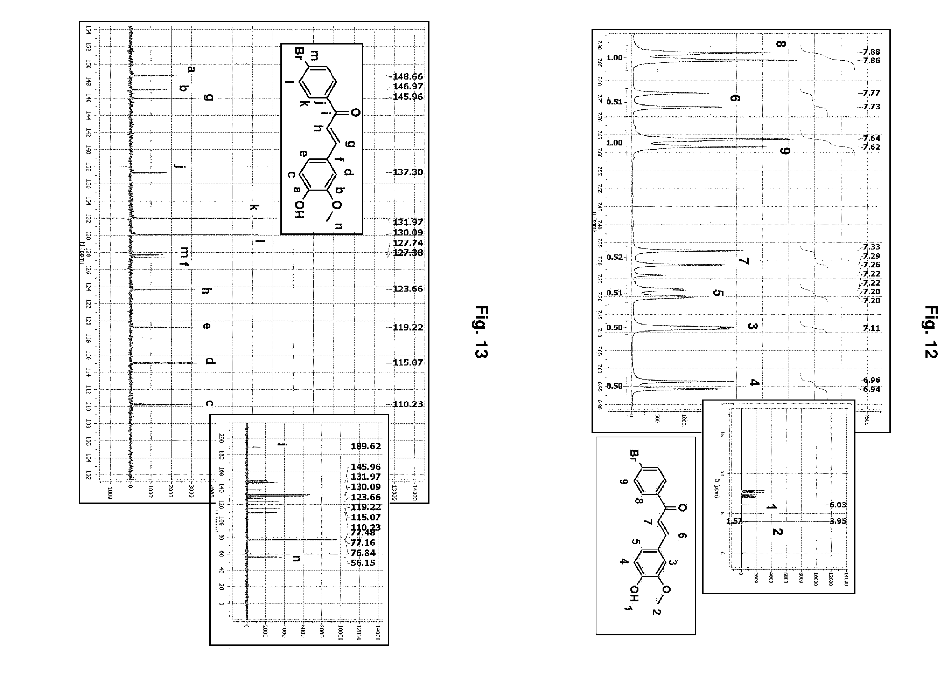

The nuclear magnetic resonance spectra of the compounds ¹H and ¹³C were characterized, the chemical displacements (ppm) of the functional groups characteristic to the structure of the compounds are shown in **Tables 5** and **6,** and the resonance spectra of ¹H and ¹³C for compounds [b], [e] and [I] with the respective chemical displacement assigned to each hydrogen and carbon of the molecule are shown in **Figures 8** to **13****.**

**Table 5: Chemical displacements determined for 1, 3, 4-thiadiazole alkylamide derivatives**

| **Compound** | **NMR-¹H (ppm)** | **NMR-¹³C (ppm)** |
|---|---|---|
| R-(CH₂)₇CH₃-R¹-H | H-Aliphatic: 0,82-0,86 ppm (t, J= 6,9 Hz, 3H); 1,50-1,24 (m, 10H); 1,87-1,79 (m, 2H); 2.77-2.81 (t, 7,7 Hz, 2H). | C-Amide: 172,39 |
| | | C-Heterocycle: 163,34, 159,87. |
| | | C-Aliphatic: 36,87; 32,20; 29,74; 29,66; 29,57; 25,87; 23,05; 14,46. |
| **(a)** | H-Amide: 12,56 (s, NH). | |
| R-(CH₂)₇CH₃-R¹-I | H-Aliphatic: 0,85-0,82 (t, J= 6,6 Hz, 3H); 1,35-1,24 (m, 8H); 1,42-1,46 (m, 2H); 1,80-1,84 (m, 2H); 2,78-2,74 (t, 6,6 Hz, 2H); | C-Amide: 172,47 |
| | | C-Heterocycle:161 ,75; 160,29 |
| | | C-Aliphatic: 36,91, 32,26; 29,78; 29,71; 29,62; 25,86; 23,11, 14,53. |
| **(b)** | H-Hydroxyl: 9,77 (s) | |
| | H-Amide: 12,77 (s, NH). | |
| R-(CH₂)₆CH₃ R¹-H | H-Aliphatic: 0,78-0,82 (t, J=6,7 Hz, 3H); 1,20-1,22 (m, 8H); 1,53-1,57 (m, 2H); 2.41-2.45 (t, J= 7,4 Hz, 2H) | C-Amide: 171,49 |
| | | C-Heterocycle:162,00; 157,46 |
| **(c)** | H-Hydroxyl: 9,7 (s) H-Amide: 12,48 (s, NH). | C- Aliphatic: 34,87; 31,13; 28,49; 28,39; 24,65; 22,08; 13,96. |
| R-(CH₂)₆CH₃ R¹ I | H-Aliphatic: 0,87-0,84 (t, J= 6,9 Hz, 3H); 1,27-1,25 (m, 8H); 1,63-1,59 (m, 2H), 2.20-2.16 (t, J= 7,4Hz, 2H) | C-Amide: 172,54 |
| | | C-Heterocycle: 163,85; 158,44 |
| | | C- Aliphatic: 32,75; 31,13; 28,45; 28,35; 24,62; 22,06; 13,97 |
| **(d)** | H-Hydroxyl: 10,04 (s) | |
| | H- Amide: 12,57 (s, NH). | |
| R-(CH₂)₅CH₃ R¹-H | H-Aliphatic: 0,31-0,35 (t, J= 6,5 Hz, 3H), 0,74 (m, 6H); 1,05-1,10 (m, 2H), | C-Amide: 171,51 |
| | | C-Heterocycle: 162,05; 157,49 |
| **(e)** | 1.98-1.95 (t, J=7,4 Hz, 2H) | C- Aliphatic: 34,91; 30,99; 28,23; 24,64; 22,01; 13,96. |
| | H-Amide: 12,02 (s, NH). | |
| R-(CH₂)₅CH₃ R¹-I | H-Aliphatic: 0,77-0,81 (t, J=6,6Hz, 3H), 1,20 (m, 6H), 1,50-1,56 (m, 2H); 2,40-2,43 (t, J=7,5Hz, 2H). | C-Amide: 171,56 |
| | | C-Heterocycle: 160,48; 157,91 |
| **(f)** | H-Hydroxyl: 10,13 (s) | C-Aliphatic: 34,89; 30,95; 28,19, 24,60; 21,97; 13,95 |
| | H-Amide: 12,51 (s, NH). | |
| R-(CH₂)₄CH₃ R¹-H | H-Aliphatic: 0,82-0,86 (t, J=6,8 Hz, 3H), 1,24-1,26 (m, 4H), 1,55-1,62 (m, 2H), 2.43-2.47 (t, J=7,5 Hz, 2H) | C-Amide: 171,46 |
| | | C-Heterocycle: 161,98, 157,47 |
| **(g)** | H-Hydroxyl: 9,66 (s) | C-Aliphatic: 34,83; 30,72; 24,32; 21,83; 13,83. |
| | H-Amide: 12,49 (s, NH). | |
| R-(CH₂)₄CH₃ R¹-I | H-Aliphatic: 0,72-0,74 (t, J= 6,8Hz, 3H), 1,15-1,18 (m, 4H), 1,47-1,52 (m, 2H), 2.34-2.37 (t, J=7,4Hz, 2H) | C-Amide: 171,54 |
| | | C-Heterocycle:160,47; 157,74 |
| **(h)** | H-Hydroxyl: 10,07 (s) | C-Aliphatic: 34,86; 30,74; 24,34; 21,86; 13,85 |
| | H-Amide: 12,45 (s, NH). | |

**Table 6: Chemical displacements determined for chalcone derivatives**

| **Compound** | **NMR ¹H (ppm)** | **NMR¹³C (ppm)** |
|---|---|---|
| 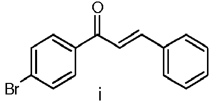 | Hβ: 7,80-7,84 (d, J=15,7 Hz) | C=O: 186,21 |
| | | Cβ: 145,60 |
| | Hα: 7,46-7,50 (d, J=15,7 Hz) | Cα: 121,63 |
| 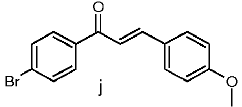 | Hβ: 7,7-7,81 (d, 15,7Hz) | C=O: 189, 56 |
| | | Cβ: 145,44 |
| | Hα: 7,37-7,33 (d, J=15,6Hz) | Cα: 119,28 |
| 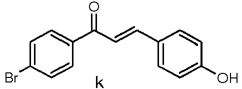 | Hβ: 7,65-7,70 (d, J= 20 Hz) | C=O: 188,09 |
| | | Cβ: 145,09 |
| | Hα: 7,64-7,69 (d, J= 20 Hz) | Cα: 118,13 |
| 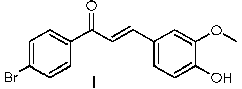 | Hβ: 7,73-7,77 (d, J=15,6Hz) | C=O: 189,62 |
| | | Cβ: 145,96 |
| | Hα: 7,29-7,33 (d, J=15,6Hz) | Cα: 119,22 |
| 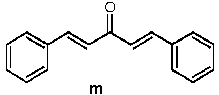 | | C=O 189,04 |
| | Hβ: 7,77-7,73 (d, J=16 Hz) | Cβ: 143,44 |
| | Hα: 7,08-7,12 (d, J= 16Hz) | Cα: 125,53 |
| 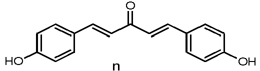 | | C=O 197,78 |
| | Hβ: 7,43-7,48 (d, J= 16Hz) | Cβ: 143,56 |
| | Hα: 6,51-6,55 (d, J= 16Hz) | Cα: 124,09 |
| 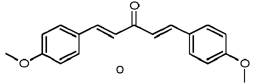 | | C=O 188,80 |
| | Hβ: 7,67-7,71 (d, J= 16 Hz) | Cβ: 142,65 |
| | Hα: 6,92-6,96 (d, J=16Hz) | Cα: 123,46 |
| 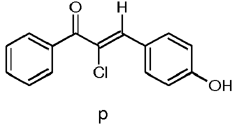 | | C=O: 192,45 |
| | Hβ: 7,45 | Cβ: 141,30 |
| | | Cα: 125,86 |
| 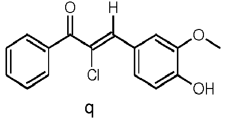 | Hβ: 7,47 | C=O: 191,86 |
| | | Cβ: 141,05 |
| | | Cα: 126,94 |

### Example 3: Physiological characterization

### Biological Assays

The synthesized compounds were evaluated in HEK-293T cell cultures transfected with cDNA from rat TRPV receptors. The compound activity was based on the capsaicin and temperature activation mechanism of the TRPV-1 receptor, which raised the levels of intracellular calcium as a consequence of the opening of the ionic channel of the receptor. The influx of calcium was measured with a fluorescent probe, Fluo 4-AM, as in the presence of an antagonist the calcium influx is blocked by the inactivation of the TRPV-1 receptor and cellular fluorescence decreases.

### Cell Culture

The HEK-293T cells were cultured in a DMEM medium supplemented with 5% fetal bovine serum, 50U/ml penicillin, 50mg/ml streptomycin and 2mM L-glutamine. The cultures were kept at 37°, in a 5% CO₂ environment, with 80% relative humidity.

### DNA Transfection

The HEK-293T cells were transfected with rat TPRV-1 pcDNA3, 97 µL of Opti-MEM and 3µL of Lipofectamine 2000 were added to a reaction tube and to a second tube 1µg of DNA and one volume of Opti-MEM were added to complete 100µL. Both tubes were incubated for 10 minutes, after which they were mixed and incubated for another 15 minutes.

### Solutions Used with the Assayed Compounds

The compounds destined to be used in the screening assays on TRPV-1 receptors were prepared in a mother solution of 10,000X in DMSO, ethanol or isopropanol and afterwards diluted for the assay in a Ringer medium until reaching a concentration of 1µM.

Solutions of capsaicin, BCTC, Lanthanum, and Ruthenium Red were used as controls at final concentrations of 0.031 µM, 1 µM, 100 µM and 10 µM, respectively.

### Treatment with FLUO-4AM

After 48 hours of transfection the cells were treated with 200 µL of trypsin, left to incubate for 5 minutes at room temperature, then gently pipetted and transposed to a 15ml tube. After being centrifuged at a speed of 6,000rpm for 10 minutes, the cells were re-suspended in 1 ml of Ringer medium, adding 1 µl of Fluo-4AM 1000X, and incubated at 37ºC for half an hour. After being centrifuged and washed with pH 7.34 PBS 1X to remove the excess probe the cell pellet was re-suspended in 1.5ml of Ringer medium.

### Activation of TRPV-1 Receptor by Capsaicin Assay

Once the rat TRPV-1 receptor had been transfected, Fluo-4 AM charged HEK 293T cells were plated out in 48-well plates at a cellular density of 50,000 cells per well, with a transfection percentage of 40-50%. Subsequently, 2 µL/well of each assayed compound and 2 µL/well of a capsaicin solution 0.031µM in 0.1 % ethanol were added. The cells were left to incubate for a period of 2-5 minutes at room temperature, after which fluorescence measurements were taken. These measurements were effected before adding the capsaicin and after adding the capsaicin. The fluorescence measurements were taken in real time on a PCR system every ten seconds in 13 cycles at 25°C. The normalized data were expressed as changes in fluorescence in response to capsaicin in time, shown in **Figures 14** to **17****.** The average values ± standard deviations obtained from the execution of two independent experiments were graphed.

### Activation of TRPV-1 Receptor by Temperature Assay

Once the rat TRPV-1 receptor had been transfected, Fluo-4 AM charged HEK 293T cells were plated out in 48-well plates at a cellular density of 50,000 cells per well, with a transfection percentage of 40-50%. Subsequently 2 µL/well of each assayed compound were added and the cells left to incubate at room temperature for 5-10 minutes before the execution of the experiment by temperature. The fluorescence measurements were then read on a real time PCR system that allows an increment of 1°C every 35 seconds from 36°C to 55°C for compounds and from 22-56 °C for transfected cells in absence of compounds. Reading of the fluorescence measurement was done using a FAM filter (483-533nM). The fluorescence data were obtained for each temperature and each compound, and have been corrected subtracting the cellular background and the base line corresponding to the fluorescence at the start of the ramp (22 or 36°C) for each sample. The values were then normalized, the data being expressed as relative fluorescence, which is the ratio between the fluorescence variation and the base line. The results were graphed in **Figures 18** to **21** as the average ± standard deviation at a temperature of 54º C obtained in two independent experiments.

### Dose Response Curve

An assay was carried out on temperature activation of the TRPV-1 receptor by incubating the transfected and Fluo-4AM charged HEK-293T cells in the presence of different concentrations of each compound assayed: 1 µM; 0.1 µM; 0.01 µM; 0.003 µM; 0.001 µM; 0.0003 µM, 0.0001 µM y 0.00001 µM. The relative fluorescence data obtained at 50°C was then used to calculate the fluorescence ratio, which is presented as normalized in relation to the relative fluorescence at initial concentration. In this way, the fluorescence ratios obtained through two independent experiments were averaged and expressed with the respective standard deviation.

The dose response curves are presented in **Figures 22** and **23****,** in which the fluorescence ratio versus the logarithmic molar concentration were adjusted to a 4 parameter sigmoidal inhibition curve, determining the respective values corresponding to the half maximal inhibitory concentration (IC₅₀) for each of the evaluated compounds. These values are shown for both groups of compounds, in **Tables 7** and **8.**

**Table 7: Values of IC₅₀ of 1, 3, 4-thiadiazol alkylamide derivatives and control antagonist BCTC.**

| **Compound** | **IC₅₀(nM)** |
|---|---|
| a | 0,046 ± 0,004 |
| b | 0,145 ± 0,051 |
| d | 1,092 ± 0,497 |
| e | 1,263 ± 0,009 |
| h | 0,298 ± 0,026 |
| BCTC | 2,120 ± 0,354 |

**Table 8: Values of IC₅₀ of compounds derived from chalcones or unsaturated alpha, beta carbonyl derivatives and control agonist BCTC.**

| **Compound** | **IC₅₀(nM)** |
|---|---|
| j | 3,892 ± 0,225 |
| I | 1,016 ± 0,206 |
| m | 7,551 ± 1,898 |
| n | 0,184 ± 0,023 |
| q | 0,257 ± 0,016 |
| BCTC | 2,120 ± 0,354 |

## Claims

1. Compounds derived from 1, 3, 4-thiadiazol alkylamides CHARACTERIZED because they inhibit TRPV-1 receptor activation.

2. Compounds derived from 1, 3, 4-thiadiazol alkylamides, according to claim 1, CHARACTERIZED because they inhibit the capsaicin and temperature activation of the TRPV-1 receptor.

3. Compounds derived from 1, 3, 4-thiadiazol alkylamides, according to claim 1, CHARACTERIZED because their R structure is a saturated alkyl chain defined by saturated linear chains of 6 to 8 carbons where X is defined as a H atom or an iodine atom.

4. Compounds derived from 1, 3, 4-thiadiazol alkylamides, according to claim 1, CHARACTERIZED because they are obtained through O-acylation reaction, amine condensation, oxidative cyclization of thiosemicarbazone, N-acylation reaction and alkaline hydrolysis.

5. Compounds derived from 1, 3, 4-thiadiazol alkylamides, according to claim 1, CHARACTERIZED because they correspond specifically to the following compounds:
a) N-[5-(4-hydroxy-5-methoxy-3-iodophenyl)-1,3,4-thiadiazole-2-yl] nonamide,
b) N-[5-(4-hydroxy-3-methoxyphenyl)-1, 3, 4- thiadiazole-2-yl]-nonamide,
c) N-[5-(4-hydroxy-5-methoxy-3-iodophenyl)-1,3,4-thiadiazole-2-yl]-octanamide,
d) N-[5-(4-hydroxy-3-methoxyphenyl)-1, 3, 4- thiadiazole-2-yl]-octanamide,
e) N-[5-(4-hydroxy-5-methoxy-3-iodophenyl)-1,3,4-thiadiazole-2-yl]-heptanamide,
f) N-[5-(4-hydroxy-3-methoxyphenyl)-1, 3, 4- thiadiazole-2-yl]-heptanamide,
g) N-[5-(4-hidroxy-5-methoxy-3-iodophenyl)-1,3,4-thiadiazole-2-yl]-hexanamide, and
h) N-[5-(4-hydroxy-3-methoxyphenyl)-1, 3, 4- thiadiazole-2-yl]-hexanamide.

6. Compounds derived from chalcones, CHARACTERIZED because they inhibit the activation of the TRPV-1 receptor.

7. Compounds derived from chalcones, according to claim 6, CHARACTERIZED because they inhibit the activation of the TRPV-1 receptor using capsaicin and temperature.

8. Compounds derived from chalcones, according to claim 6, CHARACTERIZED because their R structure can correspond to different substituent groups, such as
R1: hydrogen, a methoxyl group or a hydroxyl group;
R2: hydrogen or a methoxyl group;
R3: hydrogen or a chlorine group;
R4: hydrogen or a bromine group; and
R5: hydrogen, a methoxyl group or a hydroxyl group;

9. Compounds derived from chalcones, according to claim 6, CHARACTERIZED because the synthesis method consisted in the application of Claisen-Schmidt condensation between different benzaldehydes and acetophenones.

10. Compounds derived from chalcones, according to claim 6, CHARACTERIZED because they correspond specifically to the following compounds:
a) (*2E)*-1-(4-bromodiphenyl)-3-phenylprop-2-en-1-one,
b) *(2E)*-1-(4-bromodiphenyl)-3-(4-methoxyphenyl)prop-2-en-1-one,
c) *(2E)*-1-(4-bromodiphenyl)-3-(4-hydroxyphenyl)prop-2-en-1-one,
d) *(2E)*-1-(4-bromodiphenyl)-3-(4-hydroxy-3-methoxyfenyl)prop-2-en-1-one,
e) *(1E, 4E)*-1,5-diphenylpenta-1,4-dien-3-one,
f) *(1E, 4E)-*1,5-(4-hydroxydiphenyl)penta-1,4-dien-3-one,
g) *(1E, 4E)*-1,5-(4-methoxydiphenyl)penta-1,4-dien-3-one,
h) *(2E)*-2chloro-3-(4-hydroxyphenyl)-1-phenylprop-2-en-1-one,
i) *(2E)*-2 chloro-3-(4-hydroxy-3-methoxyphenyl)-1-phenylprop-2-en-1-one.

11. Compounds derived from 1, 3, 4-thiadiazole alklyamides and chalcones, according to claims 1 and 6, CHARACTERIZED because they have potent antagonistic activity for the temperature and capsaicin activation mode of the TRPV-1 receptor.

12. Compounds derived from 1, 3, 4-thiadiazole alklyamides and chalcones, according to claims 1 and 6, CHARACTERIZED because they present antagonistic activity in the nanomolar order, with IC₅₀ ranges between 0,04 and 1,2 nM.

13. Compounds derived from 1, 3, 4-thiadiazole alklyamides and chalcones, according to claims 1 and 6, CHARACTERIZED because they present a different binding mode on the orthosteric site of the TRPV-1 receptor, which contributes to the increase in antagonistic activity.

14. Compounds derived from 1, 3, 4-thiadiazole alklyamides and chalcones, according to claims 1 and 6, CHARACTERIZED because they exhibit antagonistic activity on TRPV-1 receptors with an increased conformational restriction, which is regulated through the isosteric replacement of the amide group present in capsaicin analogues by the 1, 3, 4-thiadiazole heterocycle, and through the double unsaturated bond and the elimination of the spacer groups in the case of chalcones.

15. Compounds derived from 1, 3, 4-thiadiazole alklyamides and chalcones, according to claims 1 and 6, CHARACTERIZED because the imposed conformational restriction allows the stabilization of the conformational state of the TRPV-1 receptor responsible for its inactivation.

16. Use of compounds derived from 1, 3, 4-thiadiazole alklyamides and chalcones, according to claims 1 and 6, CHARACTERIZED because they are used for the preparation of medication to treat diseases with TRPV-1 receptor overexpression, for example, chronic pain.
